# EUROPEAN PATENT APPLICATION

(11) **EP 4 778 538 A2**
(43) Date of publication of application: **22.07.2026**
(21) Application number: 26183538.3
(22) Date of filing: 23.09.2021
(51) Int. Cl.: A61K 47/60

(54) **COMPOUND FOR THE SEQUESTRATION OF UNDESIRABLE ANTI-PEG ANTIBODIES IN A PATIENT**

(30) Priority: 23.09.2020 EP 20197699; 07.04.2021 EP 21167124
(62) Divisional of application: 21778153.3
(71) Applicant: Ablevia biotech GmbH, 1030 Vienna (AT)
(72) Inventor: SMRZKA, Oskar, 1030 Wien (AT); WANKO, Bettina, 1030 Wien (AT)
(74) Representative: SONN Patentanwälte GmbH & Co KG

(57) **Abstract**

The present invention provides a compound for the sequestration of undesirable anti-polyethylene glycol (PEG) antibodies interfering with therapy by PEGylated active agents (such as PEGylated enzymes or antibodies) in a patient. The compound comprises an inert biopolymer scaffold and one or more PEG chains. Also provided are pharmaceutical compositions comprising the compound, as well as a method of sequestering one or more anti-PEG antibodies present in an individual and a method of inhibiting an immune reaction to a treatment with a PEGylated active agent.

## Description

The field of present invention relates to compounds for the sequestration of undesirable anti-polyethylene glycol (anti-PEG) antibodies in an individual.

PEG is a versatile, highly flexible, hydrophilic polymer comprised of repeating ethylene glycol polyether subunits that is used for many different products and applications. PEG is extensively used in the cosmetics industry, for liposome- and nanoparticle-based products and for a broad spectrum of biological applications. It is also widely used in medicine, e.g. as a laxative, for colonoscopy or as an excipient and solubilizer for pharmaceutical products.

More recently, PEG has also proven itself as an extremely useful chemical entity allowing for functional modifications of modern biopharmaceutical products: PEG can be covalently attached to a drug ("PEGylation"), thereby modifying its pharmacological and formulation properties. PEGylation changes several biological, pharmacological and biophysical parameters including stability, metabolism, solubility, adsorption, pharmacokinetics and pharmacodistribution. PEGylation is also used to mask a drug against humoral and cellular immunogenicity which is particularly important for large protein drugs containing non-human amino acid sequences. PEG is also used for controlled release and for reducing drug injection volumes.

Although PEG is *per se* a non-immunogenic, well-tolerated chemical entity, it is known that a considerable portion of the population in the industrialized world carries transient or persistent anti-PEG antibodies (Garay et al., 2012; Yang et al., 2016; Lubich et al., 2016). Anti-PEG antibodies exist not only in patients that have received treatment with a PEGylated drug, but also in up to 25% of healthy blood donors compared to 0.2% twenty years ago (reviewed by Armstrong, 2009). The observed increase of anti-PEG antibodies in the population may in part by attributed to the improvement of analytical methods and thereby distort our view (Yang et al., 2016), but the contribution of the extensive exposure to PEG and PEG-containing therapeutic compounds and vaccines, in cosmetics, processed food products or yet undefined sources remains to be determined.

Anti-PEG antibodies can be of different isotypes (predominantly IgM and IgG; see Yang et al., 2016, Lubich et al., 2016) and they can preferentially target different portions of PEG, methoxy-PEG or OH-PEG (Sherman et al., 2012; Saifer et al., 2014).

The presence of anti-PEG antibodies leads to drug neutralization and, most importantly, to a phenomenon called accelerated blood clearance (ABC). Well-known examples for the development of ABC are treatments with PEG-asparaginase (PEG-ASNase; Oncaspar^{®}) used for the treatment of acute lymphoblastic leukemia, PEG-uricase (Pegloticase; Krystexxa^{™}; used in patients with chronic gout), PEG-IFN-a2b (PegIntron^{®}) and PEG-IFN-a2a (Pegasys^{®}) used for the treatment of Hepatitis C and PEG-G-CSF (Neulasta^{®}; used for Neutropenia treatment). The immunogenicity and interaction of Polymeric-micelle carrier systems has been extensively reviewed by Shiraishi (Shiraishi et al., 2019).

The majority of PEGylated drugs are protein-based biologics (including antibodies, Fab fragments, enzymes, growth factors and cytokines, etc.). However, ABC or neutralizing anti-PEG antibodies can also occur with PEGylated peptides, PEGylated aptamers, PEGylated small organic molecules or PEG-liposomes and micelles- and nanoparticle-based drugs and vaccines after repeated injection (Park et al., 2019; Garay et al., 2012). In addition, incorporation of PEG into vaccine carrier systems has also been suggested for a lipoprotein carrier (Sekiya et al., 2017) or for polysaccharide conjugate vaccines (Zhang et al., 2015). As another example, it was demonstrated that viral gene therapy vectors could also be effectively shielded from induced or natural antibodies or other plasma proteins using PEG (Krutzke et al., 2016). Together, this points out the broad applicability and the importance of PEG for many therapeutic principles and indication fields.

Further PEGylated proteins are for instance disclosed in Akbarzadehlaleh et al., 2016, Yoshimoto et al., 2013, Gaspar et al., 2012, Kim et al., 2012, Siekmann et al., 2020, and Chapman et al., 2002. WO 2019/226538 A1 discloses selective T_{reg}

stimulator compositions with PEGylated Interleukin-2. Sharp et al, 1986, relates to the synthesis and application of a PEG-antibody affinity ligand for cell separations in aqueous polymer two-phase systems.

WO 03/040211 A2 concerns branched PEG polymers and conjugates with these branched polymers. Example 6 discloses di-PEGylated lysozyme and tri-PEGylated lysozyme.

US 2004/062748 A1 discloses PEG conjugates which are asserted to have decreased antigenicity, in particular by having hydroxyl-terminated PEG chains. One of the PEGylated proteins disclosed is porcine uricase.

To date, PEGylated drugs serve mainly the cancer therapy market (>60%), followed by a heterogenous mixture of indications including gout, Hemophilia and Hepatitis. An overview with exemplary applications and currently approved drug products is provided in Table 1 below and reflects the diversity of applications.

**Table 1 Currently approved PEGylated active agents**

| **Products** | **INN** | **Indications** |
|---|---|---|
| Adagen | Pegademase | Severe combined immunodeficiency |
| Adynovate/Adynovi, Jivi | Rurioctocog alfa pegol | Hemophilia A |
| Asclera | lauromacrogol | varicose veins |
| Cimzia | Certolizumab pegol | Psoriatic Arthritis, Crohn's Disease, Rheumatoid Arthritis and Ankylosing Spondylitis |
| Empaveli | pegcetacoplan | paroxysmal nocturnal hemoglobinuria |
| Esperoct | turoctocog alfa pegol | Hemophilia A |
| Krystexxa | Pegloticase | Chronic Gout |
| Lipodox, Doxil | doxorubicin hydrochloride | Ovarian cancer, AIDS-related Kaposi Sarcoma and Multiple Myeloma |
| Macugen | pegaptanib sodium methoxy | Wet Age-related Macular Degeneration |
| Mircera | polyethylene glycol-epoetin beta | Renal Anemia, Anemia associated with chronic kidney disease |
| Movantik | naloxegol | Non-cancer opioid-induced constipation |
| | pegfilgrastim | Chemotherapy-induced neutropenia, prophylaxis and nonmyeloid malignancies |
| Neulasta, UDENYCA, Fulphila | | |
| Omontys | peginesatide | anemia |
| Oncaspar, Asparlas | pegaspargase | Acute Lymphoblastic Leukemia |
| Palynziq | pegvaliase | phenylketonuria |
| Pegasys | peginterferon alfa-2a | hepatitis B and C |
| Peglntron | Peginterferon alfa-2b | melanoma, Hepatitis C |
| Plegridy | peginterferon beta-1a | multiple sclerosis |
| Rebinyn | nonacog beta pegol | Hemophilia B |
| Revcovi, ADA-SCID | Elapegademase | Adenosine Deaminase Severe Combined Immune Deficiency (ADA-SCID) |
| Somavert | pegvisomant | Acromegaly |
| Sylatron | Peginterferon alfa-2b | melanoma |
| Udenyca | pepfilgrastim | infection during chemotherapy |
| Ziextenzo | pegfilgrastim | infection during chemotherapy |

The emergence of anti-PEG-antibodies poses a serious problem for PEG-based active agents (i.e. pharmaceutical products, vaccines or vectors, including PEGylated proteins, peptides, oligonucleotides, small organic molecules, nanoparticles, lipid- or polysaccharide-containing drugs etc.) which remains to be solved. To date, there is no practicable protocol or therapeutic method to avoid, to remove or to neutralize anti-PEG antibodies before applying a PEGylated or a PEG-containing pharmaceutical product. Anti-PEG antibodies cause accelerated drug clearance and drug neutralization which applies also for PEGylated vaccines and gene therapy vectors. This has become an issue for particular protein drugs, such as the therapeutic enzymes PEG-asparaginase (used for ALL treatment) or PEGylated uricase (used in severe, treatment-refractory gout). In addition, PEGylated aptamers, liposomes or nanoparticles were extensively studied in preclinical models and clinically. Recent reviews are provided by Hoang Thi et al. and Abu Lila et al. (Hoang Thi TT et al., 2020; Abu Lila 2018, Book Chapter [doi.org/10.1016/B978-0-08-101750-0.00003-9]). The consequences of anti-PEG antibodies include altered pharmacokinetics and biodistribution profiles for PEGylated drugs. Anti-PEG antibodies exist in both treated and naive individuals (or individuals that have been otherwise exposed to PEG).

There is an urgent need for a method or a therapeutic agent that can remove or inactivate this special type of drug-neutralizing antibodies in the blood circulation, since anti-PEG-antibodies negatively affect the entire market for PEGylated or PEG-containing drugs, vaccines or gene therapy vectors. Thus, anti-PEG mediated clearance of PEGylated or PEG-containing active agents has become a major challenge in the field.

It was proposed to block or remove anti-PEG anti-drug antibodies by applying 2.2 mg/kg of 10kDa PEG or 550 mg/kg 20 kDa or 40 kDa PEG, prior to administering a PEGylated drug (McSweeney et al., 2019). It was found that the lower molecular weight PEG failed to restore circulation of the PEGylated active agent beyond a few hours, whereas the higher molecular weight PEGs were asserted to be effective in restoring the activity of the active agent. However, the dosing used was extremely high and it even turned out that a dose of 550 mg/kg body weight induced adverse induction of anti-PEG antibodies. WO 2019/046185 A1 (also by McSweeney et al.) discloses a similar approach based on high molecular weight free PEG.

It is thus an object of the present invention to provide compounds and methods for reducing, depleting or sequestering anti-PEG antibodies which do not have one or more of the shortcomings described above and/or with improved efficacy or safety.

The present invention provides a compound (for intracorporeal sequestration (or intracorporeal depletion) of at least one anti-PEG antibody in an individual) comprising
- a biopolymer scaffold and
- one or more PEG chains.

Furthermore, the present invention provides a pharmaceutical composition comprising the compound and at least one pharmaceutically acceptable excipient. Preferably, this pharmaceutical composition is for use in therapy.

In another aspect, the present invention provides a method of sequestering (or depleting) one or more antibodies present in an individual, comprising obtaining a pharmaceutical composition as defined herein, the composition being non-immunogenic in the individual, where the one or more antibodies are anti-PEG antibodies; and administering the pharmaceutical composition to the individual.

In yet another aspect, the present invention relates to a pharmaceutical composition, comprising the compound defined herein and further comprising an active agent and optionally at least one pharmaceutically acceptable excipient. The active agent preferably is a vector, a virus-based vaccine carrier or a protein or peptide, especially selected from the group of enzymes, enzyme inhibitors, antibodies, antibody fragments, antibody mimetics, antibody-drug conjugates, hormones, growth factors, clotting factors and cytokines, or a nucleic acid-lipid particle, a nucleic acid-polymer particle, a nucleic acid-lipid-polymer particle, or a nucleic acid.

In even yet another aspect, the present invention provides a method of inhibiting an immune reaction to a treatment with an active agent in an individual in need of treatment with the active agent, comprising obtaining said pharmaceutical composition comprising the compound and the active agent; wherein the compound of the pharmaceutical composition is non-immunogenic in the individual, and administering the pharmaceutical composition to the individual.

In further aspects the present invention provides a compound comprising a biopolymer scaffold and one or more modifications selected from the group of PEGylation, XTENylation, PASylation, methylation, glycosylation, and polysialylation. Further, the present invention provides a pharmaceutical composition comprising this compound and at least one pharmaceutically acceptable excipient. Preferably, this pharmaceutical composition is for use in therapy. The present invention also provides a method of sequestering (or depleting) one or more antibodies present in an individual, comprising obtaining this pharmaceutical composition, the composition being non-immunogenic in the individual, where the one or more antibodies are specific for the one or more modifications; and administering the pharmaceutical composition to the individual. The present invention also relates to a pharmaceutical composition, comprising this compound and further comprising an active agent and optionally at least one pharmaceutically acceptable excipient. The present invention further provides a method of inhibiting an immune reaction to a treatment with an active agent in an individual in need of treatment with the active agent, comprising obtaining said pharmaceutical composition comprising the compound and the active agent; wherein the compound of the pharmaceutical composition is non-immunogenic in the individual, and administering the pharmaceutical composition to the individual.

In the course of the present invention, it was surprisingly found that the biopolymer scaffolds used herein are particularly effective in reducing titres of undesired antibodies in an individual. Moreover, compared to high-molecular-weight and high-dose free PEG which carries a considerable risk of immunogenicity (which is exactly the opposite of what is intended with an anti-PEG antibody-reducing agent) the compound of the present invention has a more favourable safety profile, in particular it turned out to be non-immunogenic. In addition, it was unexpectedly found that PEG moieties of 10 kDa or less were more effective than higher molecular weight PEGs (see example section).

The detailed description given below relates to all of the above aspects of the invention unless explicitly excluded.

In general, antibodies are essential components of the humoral immune system, offering protection from infections by foreign organisms including bacteria, viruses, fungi or parasites. However, under certain circumstances - including autoimmune diseases, organ transplantation, blood transfusion or upon administration of biomolecular drugs or gene delivery vectors - antibodies can target the patient's own body (or the foreign tissue or cells or the biomolecular drug or vector just administered), thereby turning into harmful or disease-causing entities. Certain antibodies can also interfere with probes for diagnostic imaging. In the following, such antibodies are generally referred to as "undesired antibodies" or "undesirable antibodies".

With few exceptions, selective removal of undesired antibodies has not reached clinical practice. It is presently restricted to very few indications: One of the known techniques for selective antibody removal (although not widely established) is immunoapheresis. In contrast to immunoapheresis (which removes immunoglobulin), selective immunoapheresis involves the filtration of plasma through an extracorporeal, selective antibody-adsorber cartridge that will deplete the undesired antibody based on selective binding to its antigen binding site. Selective immunoapheresis has for instance been used for removing anti-A or anti-B antibodies from the blood prior to AB0-incompatible transplantation or with respect to indications in transfusion medicine (Teschner et al., 2012). Selective apheresis was also experimentally applied in other indications, such as neuroimmunological indications (Tetala et al.) or myasthenia gravis (Lazaridis et al.), but is not yet established in the clinical routine. One reason that selective immunoapheresis is only hesitantly applied is the fact that it is a cost intensive and cumbersome intervention procedure that requires specialized medical care. Moreover, it is not known in the prior art how to deplete undesired antibodies rapidly and efficiently.

Unrelated to apheresis, Morimoto et al. discloses dextran as a generally applicable multivalent scaffold for improving immunoglobulin-binding affinities of peptide and peptidomimetic ligands such as the FLAG peptide. WO 2011/130324 A1 relates to compounds for prevention of cell injury. EP 3 059 244 A1 relates to a C-met protein agonist.

As mentioned, apheresis is applied extracorporeally. By contrast, also several approaches to deplete undesirable antibodies intracorporeally were proposed in the prior art, mostly in connection with certain autoimmune diseases involving autoantibodies or anti-drug antibodies:
Lorentz et al. discloses a technique whereby erythrocytes are charged in situ with a tolerogenic payload driving the deletion of antigen-specific T cells. This is supposed to ultimately lead to reduction of the undesired humoral response against a model antigen. A similar approach is proposed in Pishesha et al. In this approach, erythrocytes are loaded ex vivo with a peptide-antigen construct that is covalently bound to the surface and reinjected into the animal model for general immunotolerance induction.

WO 92/13558 A1 relates to conjugates of stable nonimmunogenic polymers and analogs of immunogens that possess the specific B cell binding ability of the immunogen and which, when introduced into individuals, induce humoral anergy to the immunogen. Accordingly, these conjugates are disclosed to be useful for treating antibody-mediated pathologies that are caused by foreign- or self-immunogens. In this connection, see also EP 0 498 658 A2.

Taddeo et al. discloses selectively depleting antibody producing plasma cells using anti-CD138 antibody derivatives fused to an ovalbumin model antigen thereby inducing receptor crosslinking and cell suicide in vitro selectively in those cells that express the antibody against the model antigen.

Apitope International NV (Belgium) is presently developing soluble tolerogenic T-cell epitope peptides which may lead to expression of low levels of co-stimulatory molecules from antigen presenting cells inducing tolerance, thereby suppressing antibody response (see e.g. Jansson et al.). These products are currently under preclinical and early clinical evaluation, e.g. in multiple sclerosis, Grave's disease, intermediate uveitis, and other autoimmune conditions as well as Factor VIII intolerance.

Similarly, Selecta Biosciences, Inc. (USA) is currently pursuing strategies of tolerance induction by so-called Synthetic Vaccine Particles (SVPs). SVP-Rapamycin is supposed to induce tolerance by preventing undesired antibody production via selectively inducing regulatory T cells (see Mazor et al.).

Mingozzi et al. discloses decoy adeno-associated virus (AAV) capsids that adsorb antibodies but cannot enter a target cell.

WO 2015/136027 Al discloses carbohydrate ligands presenting the minimal Human Natural Killer-1 (HNK-1) epitope that bind to anti-MAG (myelin-associated glycoprotein) IgM antibodies, and their use in diagnosis as well as for the treatment of anti-MAG neuropathy. WO 2017/046172 Al discloses further carbohydrate ligands and moieties, respectively, mimicking glycoepitopes comprised by glycosphingolipids of the nervous system which are bound by anti-glycan antibodies associated with neurological diseases. The document further relates to the use of these carbohydrate ligands/moieties in diagnosis as well as for the treatment of neurological diseases associated with anti-glycan antibodies.

US 2004/0258683 Al discloses methods for treating systemic lupus erythematosus (SLE) including renal SLE and methods of reducing risk of renal flare in individuals with SLE, and methods of monitoring such treatment. One disclosed method of treating SLE including renal SLE and reducing risk of renal flare in an individual with SLE involves the administration of an effective amount of an agent for reducing the level of anti-double-stranded DNA (dsDNA) antibody, such as a dsDNA epitope as in the form of an epitope-presenting carrier or an epitope-presenting valency platform molecule, to the individual.

US patent no. 5,637,454 relates to assays and treatments of autoimmune diseases. Agents used for treatment might include peptides homologous to the identified antigenic, molecular mimicry sequences. It is disclosed that these peptides could be delivered to a patient in order to decrease the amount of circulating antibody with a particular specificity.

US 2007/0026396 A1 relates to peptides directed against antibodies, which cause cold-intolerance, and the use thereof. It is taught that by using the disclosed peptides, in vivo or ex vivo neutralization of undesired autoantibodies is possible. A comparable approach is disclosed in WO 1992/014150 A1 or in WO 1998/030586 A2.

WO 2018/102668 A1 discloses a fusion protein for selective degradation of disease-causing or otherwise undesired antibodies. The fusion protein (termed "Seldeg") includes a targeting component that specifically binds to a cell surface receptor or other cell surface molecule at near-neutral pH, and an antigen component fused directly or indirectly to the targeting component. Also disclosed is a method of depleting a target antigen-specific antibody from a patient by administering to the patient a Seldeg having an antigen component configured to specifically bind the target antigen-specific antibody.

WO 2015/181393 A1 concerns peptides grafted into sunflower-trypsin-inhibitor- (SFTI-) and cyclotide-based scaffolds. These peptides are disclosed to be effective in autoimmune disease, for instance citrullinated fibrinogen sequences that are grafted into the SFTI scaffold have been shown to block autoantibodies in rheumatoid arthritis and inhibit inflammation and pain. These scaffolds are disclosed to be non-immunogenic.

Erlandsson et al. discloses in vivo clearing of idiotypic antibodies with anti-idiotypic antibodies and their derivatives.

Berlin Cures Holding AG (Germany) has proposed an intravenous broad spectrum neutralizer DNA aptamer (see e.g. WO 2016/020377 A1 and WO 2012/000889 A1) for the treatment of dilated cardiomyopathy and other GPCR-autoantibody related diseases that in high dosage is supposed to block autoantibodies by competitive binding to the antigen binding regions of autoantibodies. In general, aptamers did not yet achieve a breakthrough and are still in a preliminary stage of clinical development. The major concerns are still biostability and bioavailability, constraints such as nuclease sensitivity, toxicity, small size and renal clearance. A particular problem with respect to their use as selective antibody antagonists are their propensity to stimulate the innate immune response.

WO 00/33887 A2 discloses methods for reducing circulating levels of antibodies, particularly disease-associated antibodies. The methods entail administering effective amounts of epitope-presenting carriers to an individual. In addition, ex vivo methods for reducing circulating levels of antibodies are disclosed which employ epitope-presenting carriers.

US 6,022,544 A relates to a method for reducing an undesired antibody response in a mammal by administering to the mammal a non-immunogenic construct which is free of high molecular weight immunostimulatory molecules. The construct is disclosed to contain at least two copies of a B cell membrane immunoglobulin receptor epitope bound to a pharmaceutically acceptable non-immunogenic carrier.

However, the approaches to deplete undesirable antibodies intracorporeally disclosed in the prior art have many shortcomings. In particular, neither of them has been approved for regular clinical use, let alone clinical use for sequestering anti-PEG antibodies.

The biopolymer scaffold used in the present invention may be a mammalian biopolymer such as a human biopolymer, a non-human primate biopolymer, a sheep biopolymer, a pig biopolymer, a dog biopolymer or a rodent biopolymer. In particular the biopolymer scaffold is a protein, especially a (non-modified or non-modified with respect to its amino-acid sequence) plasma protein. Preferably, the biopolymer scaffold is a mammalian protein such as a human protein, a non-human primate protein, a sheep protein, a pig protein, a dog protein or a rodent protein. Typically, the biopolymer scaffold is a non-immunogenic and/or non-toxic protein that preferably circulates in the plasma of healthy (human) individuals and can e.g. be efficiently scavenged or recycled by scavenging receptors, such as e.g. present on myeloid cells or on liver sinusoidal endothelial cells (reviewed by Sorensen et al., 2015).

According to a particular preference, the biopolymer scaffold is a (preferably human) globulin, preferably selected from the group consisting of immunoglobulins, alpha1-globulins, alpha2-globulins and beta-globulins, in particular immunoglobulin G, haptoglobin and transferrin.

The biopolymer scaffold may also be (preferably human) albumin, hemopexin, alpha-1-antitrypsin, C1 esterase inhibitor, lactoferrin or non-immunogenic (i.e. non-immunogenic in the individual to be treated) fragments of all of the aforementioned proteins, including the globulins.

In another preference, the biopolymer scaffold is an anti-CD163 antibody (i.e. an antibody specific for a CD163 protein) or CD163-binding fragment thereof.

Human CD163 (Cluster of Differentiation 163) is a 130 kDa membrane glycoprotein (formerly called M130) and prototypic class I scavenger receptor with an extracellular portion consisting of nine scavenger receptor cysteine-rich (SRCR) domains that are responsible for ligand binding. CD163 is an endocytic receptor present on macrophages and monocytes, it removes hemoglobin/haptoglobin complexes from the blood but it also plays a role in anti-inflammatory processes and wound healing. Highest expression levels of CD163 are found on tissue macrophages (e.g. Kupffer cells in the liver) and on certain macrophages in spleen and bone marrow. Because of its tissue- and cell-specific expression and entirely unrelated to depletion of undesirable antibodies, CD163 is regarded as a macrophage target for drug delivery of e.g. immunotoxins, liposomes or other therapeutic compound classes (Skytthe et al., 2020).

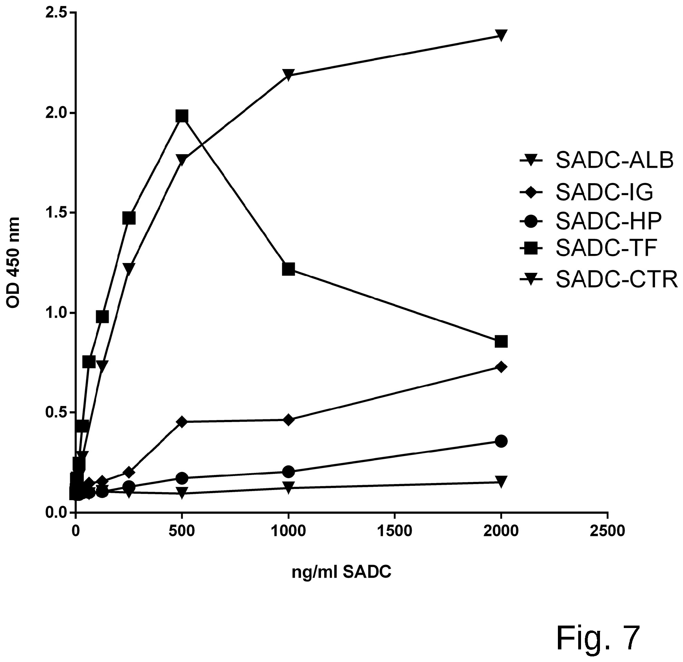

Monoclonal anti-CD163 antibodies and the SRCR domains they are binding are for instance disclosed in Madsen et al., 2004, in particular Fig. 7. Further anti-CD163 antibodies and fragments thereof are e.g. disclosed in WO 2002/032941 A2 or WO 2011/039510 A2. At least two structurally different binding sites for ligands were mapped by using domain-specific antibodies such as e.g. monoclonal antibody (mAB) EDhu1 (see Madsen et al., 2004). This antibody binds to the third SRCR of CD163 and competes with hemoglobin/haptoglobin binding to CD163. Numerous other antibodies against different domains of CD163 were previously described in the literature, including Mac2-158, KiM8, GHI/61 and RM3/1, targeting SRCR domains 1, 3, 7 and 9, respectively. In addition, conserved bacterial binding sites were mapped and it was demonstrated that certain antibodies were able to inhibit either bacterial binding but not hemoglobin/haptoglobin complex binding and vice versa. This points to different modes of binding and ligand interactions of CD163 (Fabriek et al., 2009; see also citations therein).

Entirely unrelated to depletion of undesirable antibodies, CD163 was proposed as a target for cell-specific drug delivery because of its physiological properties. Tumor-associated macrophages represent one of the main targets where the potential benefit of CD163-targeting is currently explored. Remarkably, numerous tumors and malignancies were shown to correlate with CD163 expression levels, supporting the use of this target for tumor therapy. Other proposed applications include CD163 targeting by anti-drug conjugates (ADCs) in chronic inflammation and neuroinflammation (reviewed in Skytthe et al., 2020). Therefore, CD163-targeting by ADCs notably with dexamethasone or stealth liposome conjugates represents therapeutic principle which is currently studied (Graversen et al., 2012; Etzerodt et al., 2012).

In that context, there are references indicating that anti-CD163 antibodies can be rapidly internalized by endocytosis when applied *in vivo.* This was shown for example for mAB Ed-2 (Dijkstra et al., 1985; Graversen et al., 2012) or for mAB Mac2-158 / KN2/NRY (Granfeldt et al., 2013). Based on those observations in combination with observations made in the course of the present invention (see in particular example section), anti-CD163 antibodies and CD163-binding turned out to be highly suitable biopolymer scaffolds for depletion/sequestration of undesirable antibodies.

Numerous anti-CD163 antibodies and CD163-binding fragments thereof are known in the art (see e.g. above). These are suitable to be used as a biopolymer scaffold for the present invention. For instance, any anti-CD163 antibody or fragment thereof mentioned herein or in WO 2011/039510 A2 (which is included herein by reference) may be used as a biopolymer scaffold in the invention. Preferably, the biopolymer scaffold of the inventive compound is antibody Mac2-48, Mac2-158, 5C6-FAT, BerMac3, or E10B10 as disclosed in WO 2011/039510, in particular humanised Mac2-48 or Mac2-158 as disclosed in WO 2011/039510 A2.

In a preferred embodiment, the anti-CD163 antibody or CD163-binding fragment thereof comprises a heavy-chain variable (V_{H}) region comprising one or more complementarity-determining region (CDR) sequences selected from the group consisting of SEQ ID NOs: 11-13 of WO 2011/039510 A2.

In addition, or alternatively thereto, in a preferred embodiment, the anti-CD163 antibody or CD163-binding fragment thereof comprises a light-chain variable (V_{L}) region comprising one or more CDR sequences selected from the group consisting of SEQ ID NOs: 14-16 of WO 2011/039510 A2 or selected from the group consisting of SEQ ID NOs:17-19 of WO 2011/039510 A2.

In a further preferred embodiment, the anti-CD163 antibody or CD163-binding fragment thereof comprises a heavy-chain variable (V_{H}) region comprising or consisting of the amino acid sequence of SEQ ID NO: 20 of WO 2011/039510 A2.

In addition, or alternatively thereto, in a preferred embodiment, the anti-CD163 antibody or CD163-binding fragment thereof comprises a light-chain variable (V_{L}) region comprising or consisting of the amino acid sequence of SEQ ID NO: 21 of WO 2011/039510 A2.

In a further preferred embodiment, the anti-CD163 antibody or CD163-binding fragment thereof comprises a heavy-chain variable (V_{H}) region comprising or consisting of the amino acid sequence of SEQ ID NO: 22 of WO 2011/039510 A2.

In addition, or alternatively thereto, in a preferred embodiment, the anti-CD163 antibody or CD163-binding fragment thereof comprises a light-chain variable (V_{L}) region comprising or consisting of the amino acid sequence of SEQ ID NO: 23 of WO 2011/039510 A2.

In a further preferred embodiment, the anti-CD163 antibody or CD163-binding fragment thereof comprises a heavy-chain variable (V_{H}) region comprising or consisting of the amino acid sequence of SEQ ID NO: 24 of WO 2011/039510 A2.

In addition, or alternatively thereto, in a preferred embodiment, the anti-CD163 antibody or CD163-binding fragment thereof comprises a light-chain variable (V_{L}) region comprising or consisting of the amino acid sequence of SEQ ID NO: 25 of WO 2011/039510 A2.

In the context of the present invention, the anti-CD163 antibody may be a mammalian antibody such as a humanized or human antibody, a non-human primate antibody, a sheep antibody, a pig antibody, a dog antibody or a rodent antibody. In embodiments, the anti-CD163 antibody may be monoclonal.

According to a preference, the anti-CD163 antibody is selected from IgG, IgA, IgD, IgE and IgM.

According to a further preference, the CD163-binding fragment is selected from a Fab, a Fab', a F(ab)2, a Fv, a single-chain antibody, a nanobody and an antigen-binding domain.

CD163 amino acid sequences are for instance disclosed in WO 2011/039510 A2 (which is included here by reference). In the context of the present invention, the anti-CD163 antibody or CD163-binding fragment thereof is preferably specific for a human CD163, especially with the amino acid sequence of any one of SEQ ID NOs: 28-31 of WO 2011/039510 A2.

In a further preferred embodiment, the anti-CD163 antibody or CD163-binding fragment thereof is specific for the extracellular region of CD163 (e.g. for human CD163: amino acids 42-1050 of UniProt Q86VB7, sequence version 2), preferably for an SRCR domain of CD163, more preferably for any one of SRCR domains 1-9 of CD163 (e.g. for human CD163: amino acids 51-152, 159-259, 266-366, 373-473, 478-578, 583-683, 719-819, 824-926 and 929-1029, respectively, of UniProt Q86VB7, sequence version 2), even more preferably for any one of SRCR domains 1-3 of CD163 (e.g. for human CD163: amino acids 51-152, 159-259, 266-366, and 373-473, respectively, of UniProt Q86VB7, sequence version 2), especially for SRCR domain 1 of CD163 (in particular with the amino acid sequence of any one of SEQ ID NOs: 1-8 of WO 2011/039510 A2, especially SEQ ID NO: 1 of WO 2011/039510 A2).

In a particular preference, the anti-CD163 antibody or CD163-binding fragment thereof is capable of competing for binding to (preferably human) CD163 with a (preferably human) hemoglobin-haptoglobin complex (e.g. in an ELISA).

In another particular preference, the anti-CD163 antibody or CD163-binding fragment thereof is capable of competing for binding to human CD163 with any of the anti-human CD163 mAbs disclosed herein, in particular Mac2-48 or Mac2-158 as disclosed in WO 2011/039510 A2.

In yet another particular preference, the anti-CD163 antibody or CD163-binding fragment thereof is capable of competing for binding to human CD163 with an antibody having a heavy chain variable (VH) region consisting of the amino acid sequence 
DVQLQESGPGLVKPSQSLSLTCTVTGYSITSDYAWNWIRQFPGNKLEWMGYIT
YSGITNYNPSLKSQISITRDTSKNQFFLQLNSVTTEDTATYYCVSGTYYFDYW
GQGTTLTVSS (SEQ ID NO: 1),
 and having a light-chain variable (VL) region consisting of the amino acid sequence

SVVMTQTPKSLLISIGDRVTITCKASQSVSSDVAWFQQKPGQSPKPLIYYASNRY
TGVPDRFTGSGYGTDFTFTISSVQAEDLAVYFCGQDYTSPRTFGGGTKLEIKRA (SEQ
ID NO: 2) (e.g. in an ELISA).

Details on competitive binding experiments are known to the person of skilled in the art (e.g. based on ELISA) and are for instance disclosed in WO 2011/039510 A2 (which is included herein by reference).

The epitopes of antibodies E10B10 and Mac2-158 as disclosed in WO 2011/039510 were mapped (see example section). These epitopes are particularly suitable for binding of the anti-CD163 antibody (or CD163-binding fragment thereof) of the inventive compound.

Accordingly, in particularly preferred embodiment, the anti-CD163 antibody or CD163-binding fragment thereof is specific for peptide consisting of 7-25, preferably 8-20, even more preferably 9-15, especially 10-13 amino acids, wherein the peptide comprises the amino acid sequence CSGRVEVKVQEEWGTVCNNGWSMEA (SEQ ID NO: 3) or a 7-24 amino-acid fragment thereof. Preferably, this peptide comprises the amino acid sequence GRVEVKVQEEW (SEQ ID NO: 4), WGTVCNNGWS (SEQ ID NO: 5) or WGTVCNNGW (SEQ ID NO: 6). More preferably, the peptide comprises an amino acid sequence selected from EWGTVCNNGWSME (SEQ ID NO: 7), QEEWGTVCNNGWS (SEQ ID NO: 8), WGTVCNNGWSMEA (SEQ ID NO: 9), EEWGTVCNNGWSM (SEQ ID NO: 10), VQEEWGTVCNNGW (SEQ ID NO: 11), EWGTVCNNGW (SEQ ID NO: 12) and WGTVCNNGWS (SEQ ID NO: 5). Even more preferably, the peptide consists of an amino acid sequence selected from EWGTVCNNGWSME (SEQ ID NO: 7), QEEWGTVCNNGWS (SEQ ID NO: 8), WGTVCNNGWSMEA (SEQ ID NO: 9), EEWGTVCNNGWSM (SEQ ID NO: 10), VQEEWGTVCNNGW (SEQ ID NO: 11), EWGTVCNNGW (SEQ ID NO: 12) and WGTVCNNGWS (SEQ ID NO: 5), optionally with an N-terminal and/or C-terminal cysteine residue.

Accordingly, in another particularly preferred embodiment, the anti-CD163 antibody or CD163-binding fragment thereof is specific for a peptide consisting of 7-25, preferably 8-20, even more preferably 9-15, especially 10-13 amino acids, wherein the peptide comprises the amino acid sequence DHVSCRGNESALWDCKHDGWG (SEQ ID NO: 13) or a 7-20 amino-acid fragment thereof. Preferably, this peptide comprises the amino acid sequence ESALW (SEQ ID NO: 14) or ALW. More preferably, the peptide comprises an amino acid sequence selected from ESALWDC (SEQ ID NO: 15), RGNESALWDC (SEQ ID NO: 16), SCRGNESALW (SEQ ID NO: 17), VSCRGNESALWDC (SEQ ID NO: 18), ALWDCKHDGW (SEQ ID NO: 19), DHVSCRGNESALW (SEQ ID NO: 20), CRGNESALWD (SEQ ID NO: 21), NESALWDCKHDGW (SEQ ID NO: 22) and ESALWDCKHDGWG (SEQ ID NO: 23). Even more preferably, the peptide consists of an amino acid sequence selected from ESALWDC (SEQ ID NO: 15), RGNESALWDC (SEQ ID NO: 16), SCRGNESALW (SEQ ID NO: 17), VSCRGNESALWDC (SEQ ID NO: 18), ALWDCKHDGW (SEQ ID NO: 19), DHVSCRGNESALW (SEQ ID NO: 20), CRGNESALWD (SEQ ID NO: 21), NESALWDCKHDGW (SEQ ID NO: 22) and ESALWDCKHDGWG (SEQ ID NO: 23), optionally with an N-terminal and/or C-terminal cysteine residue.

Accordingly, in another particularly preferred embodiment, the anti-CD163 antibody or CD163-binding fragment thereof is specific for a peptide consisting of 7-25, preferably 8-20, even more preferably 9-15, especially 10-13 amino acids, wherein the peptide comprises the amino acid sequence SSLGGTDKELRLVDGENKCS (SEQ ID NO: 24) or a 7-19 amino-acid fragment thereof. Preferably, this peptide comprises the amino acid sequence SSLGGTDKELR (SEQ ID NO: 25) or SSLGG (SEQ ID NO: 26). More preferably, the peptide comprises an amino acid sequence selected from SSLGGTDKELR (SEQ ID NO: 25), SSLGGTDKEL (SEQ ID NO: 28), SSLGGTDKE (SEQ ID NO: 29), SSLGGTDK (SEQ ID NO: 30), SSLGGTD (SEQ ID NO: 31), SSLGGT (SEQ ID NO: 32) and SSLGG (SEQ ID NO: 26). Even more preferably, the peptide consists of an amino acid sequence selected from SSLGGTDKELR (SEQ ID NO: 25), SSLGGTDKEL (SEQ ID NO: 28), SSLGGTDKE (SEQ ID NO: 29), SSLGGTDK (SEQ ID NO: 30), SSLGGTD (SEQ ID NO: 31), SSLGGT (SEQ ID NO: 32) and SSLGG (SEQ ID NO: 26), optionally with an N-terminal and/or C-terminal cysteine residue.

The one or more PEG chains (or PEG moieties) are preferably covalently conjugated (or covalently bound) to the biopolymer scaffold via a (non-immunogenic) linker known in the art, e.g. by means of a N-Hydroxysuccinimide (NHS) ester. For instance, NHS-functionalized PEG (PEG-NHS) is widely commercially available. Alternatively, or in addition thereto, for example a bifunctional linker (such as N-γ-maleimidobutyryl-oxysulfosuccinimide ester, GMBS) may be directly attached to the biopolymer scaffold via formation of an amide bond. Subsequently, e.g. a peptide containing a cysteine and one or more amino groups, or a cysteine on its own may be conjugated to the bifunctional linker via formation of a thioether bond. NHS-PEG, for instance, may then finally be reacted with the amino group(s) on the linker peptide (or the linker cysteine) to link the PEG chain(s) to the biopolymer scaffold.

According to a particular preference, at least a portion of the one or more PEG chains (or PEG moieties) are covalently conjugated (or covalently bound) to the biopolymer scaffold via at least one linker. Preferably, the at least one linker comprises a peptide or a single amino acid, in particular a cysteine. This is because, in the course of the present invention, it was surprisingly found that the density of modifications such as PEG on the scaffold were increased when using peptide/amino-acid linkers (see in particular Example 15).

Preferably, the biopolymer scaffold and/or the one or more PEG chains (or other modification(s)) is covalently conjugated (or covalently bound) directly to the peptide (as also shown in Example 15) or single amino acid such as cysteine. Many different coupling methods are available to the skilled person. For instance, the biopolymer scaffold and/or the one or more PEG chains may be coupled to a lysine residue, a tyrosine residue, a cysteine residue, the N-terminus or the C-terminus of the peptide (e.g., in an embodiment, the biopolymer scaffold may be coupled to the N-terminus and the one or more PEG chains to the C-terminus or vice versa). In case of a single amino acid, the N-terminus and the C-terminus may be used for coupling, as well as the side chain (e.g. the thiol group in case of cysteine). Suitable selective coupling (bioconjugation) to amino acids and peptides is reviewed in detail e.g. by Koniev and Wagner, 2015. Coupling to natural amino acids may (also) be performed via lysine, N-terminal amine, cysteine, tryptophan, tyrosine, methionine, histidine, backbone-amide or carboxylic acid. Furthermore, incorporation of unnatural amino acids and bioorthogonal chemistry by expansion of the genetic code can also be used for the peptide of the linker, as reviewed e.g. by Elia 2020.

Many different amino acid sequences and sequence lengths are suitable for the peptide of the linker (if present). The peptide may e.g. also contain unnatural amino acids or have modified side chains (e.g. with biotin). However, in any case, it is preferred that the peptide does not bind to any HLA Class I or HLA Class II molecule (i.e. of the individual to be treated, e.g. human), in order to prevent presentation and stimulation via a T-cell receptor in vivo and thereby induce an immune reaction. Therefore, to avoid T-cell epitope activity as much as possible, the peptide preferably fulfils one or more of the following characteristics:
- To reduce the probability for the peptide to bind to an HLA Class II or Class I molecule, the peptide has a preferred length of 4-8 amino acids, although somewhat shorter or longer lengths are still acceptable (in particular 2-13 amino acids, preferably 3-11 amino acids, more preferably 4-9 amino acids).
- To further reduce the probability that such a peptide binds to an HLA Class II or Class I molecule, it is preferred to test the candidate peptide sequence by HLA binding prediction algorithms such as NetMHCII-2.3 (reviewed by Jensen et al 2018). Preferably, the peptide has (predicted) HLA binding (IC50) of at least 500 nM. More preferably, HLA binding (IC50) is more than 1000 nM, especially more than 2000 nM (cf. e.g. Peters et al 2006). In order to decrease the likelihood of HLA Class I binding, NetMHCpan 4.0 may also be applied for prediction (Jurtz et al 2017).
- To further reduce the probability that such a peptide binds to an HLA Class I molecule, the NetMHCpan Rank percentile threshhold can be set to a background level of 10% according to Ko 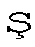 aloǧlu-Yal 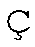 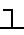 n et al 2018. Preferably, the peptide has a %Rank value of more than 3, preferably more than 5, more preferably more than 10 according to the NetMHCpan algorithm.
- To further reduce the probability that such a peptide binds to an HLA Class II molecule, it is beneficial to perform in vitro HLA-binding assays commonly used in the art such as for example refolding assays, iTopia, peptide rescuing assays or array-based peptide binding assays. Alternatively, or in addition thereto, LC-MS based analytics can be used, as e.g. reviewed by Gfeller et al 2016.

The peptides of the linker may be linear or circularized/constrained. Several common techniques are available for circularization/constraining of peptides, see e.g. Ong et al 2017 or Bozovičar et al, 2021.

As also illustrated above, it is highly preferred when the peptide of the linker (if present) is non-immunogenic and/or biologically inert in a mammal, preferably in a human, in a non-human primate, in a sheep, in a pig, in a dog or in a rodent. Preferably, such a non-immunogenic peptide has an IC50 higher than 100 nM, preferably higher than 500 nM, even more preferably higher than 1000 nM, especially higher than 2000 nM, against HLA-DRB1_0101 as predicted by the NetMHCII-2.3 algorithm. The NetMHCII-2.3 algorithm is described in detail in Jensen et al, which is incorporated herein by reference. The algorithm is publicly available on http://www.cbs.dtu.dk/services/NetMHCII-2.3/. Even more preferably, the peptide does not bind to any HLA and/or MHC molecule (e.g. in a mammal, preferably in a human, in a non-human primate, in a sheep, in a pig, in a dog or in a rodent; or of the individual to be treated) in vivo. Alternatively, or in addition thereto, computational docking analysis to avoid binding of the peptide in an MHC-I molecule cleft can be used, as exemplified by Ramana et al 2020.

Alternatively, or in addition thereto, the peptide does preferably not comprise a B-cell epitope. Suitable in-silico methods for B-cell epitope prediction such as support vector machine- or decision tree-based methods were recently reviewed e.g. by Sun et al 2019, or by Galanis et al 2021.

Since, in particular, lysine, tyrosine and cysteine residues can be used for branched linking of PEG chains (or other modifications) - i.e. a single peptide may then be able to link several PEG chains (or other modifications) to the biopolymer scaffold - it is preferred that the peptide contains at least one lysine residue, preferably at least two, more preferably at least three, even more preferably at least four, especially at least five lysine residues; and/or the peptide contains at least one tyrosine residue, preferably at least two, more preferably at least three, even more preferably at least four, especially at least five tyrosine residues; and/or the peptide contains at least one cysteine residue, preferably at least two, more preferably at least three, even more preferably at least four, especially at least five cysteine residues. To each of the

aforementioned residues, a PEG chain (or other modifications) may be bound.

Consequently, in a further preferred embodiment, at least two, preferably at least three, more preferably at least four, especially at least five PEG chains are bound to a single of the at least one linker, preferably to the peptide of the linker.

To achieve higher flexibility in the peptide of the linker, the peptide may contain at least one glycine residue, preferably at least two, more preferably at least three, even more preferably at least four, especially at least five glycine residues.

According to a further preference, the peptide of the linker has a terminal (i.e. N-terminal and/or C-terminal) cysteine residue, for more convenient coupling. By way of example, the biopolymer scaffold may be linked to a terminal cysteine of the peptide (e.g. by activation of lysine residues of the biopolymer scaffold with sulfo-GMBS) and PEG may then be linked to the free N-terminus of the peptide by incubating with NHS-PEG (see Example 15).

In a particular preferment, the peptide comprises the amino-acid sequence (X₁-(X₂)ₘ)ₙ, wherein m is an integer from 1 to 5, preferably from 2 to 4, wherein n is an integer from 1 to 5, preferably from 2 to 5. Preferably, independently for each occurrence, X₁ is lysine, tyrosine, tryptophan, methionine, histidine or cysteine, and, independently for each occurrence, X₂ is any other amino acid that is not X₁, preferably glycine.

The compound of the present invention may comprise e.g. at least two, preferably between 3 and 40 PEG chains (with the same or differing molecular weights).

Preferably, each of the PEG chains is covalently bound to the biopolymer scaffold.

In the course of the present invention, certain molecular weight ranges for the PEG chain turned out to be particularly advantageous (see also example section). Thus, in a preference, at least one, preferably at least 10%, more preferably at least 20%, even more preferably at least 40%, yet more preferably at least 60%, yet even more preferably at least 80% or even at least 90%, especially each of the one or more PEG chains have a molecular weight of 100-10000 Da, preferably 200-8000 Da, more preferably 300-6000 Da, yet more preferably 400-5000 Da, even more preferably 500-4000 Da, yet even more preferably 600-3000 Da, especially 700-2500 Da or even 1500-2500 Da.

To increase the sequestration efficiency even further, it is preferred that at least one, preferably at least 10%, more preferably at least 20%, even more preferably at least 40%, yet more preferably at least 60%, yet even more preferably at least 80% or even at least 90%, especially each of the one or more PEG chains have a free methoxy end group or a free hydroxyl end group. "Free" in this context means that this PEG end group is not covalently bound to another molecule such as e.g. a further peptide or another functional group or a protective group.

It is highly preferred when the compound of the present invention is non-immunogenic in a mammal, preferably in a human, in a non-human primate, in a sheep, in a pig, in a dog or in a rodent.

In the context of the present invention, a non-immunogenic compound preferably is a compound wherein the biopolymer scaffold (if it is a protein) have an IC50 higher than 100 nM, preferably higher than 500 nM, even more preferably higher than 1000 nM, especially higher than 2000 nM, against HLA-DRB1_0101 as predicted by the NetMHCII-2.3 algorithm. The NetMHCII-2.3 algorithm is described in detail in Jensen et al., which is incorporated herein by reference. The algorithm is publicly available under http://www.cbs.dtu.dk/services/NetMHCII-2.3/. Even more preferably, a non-immunogenic compound (or pharmaceutical composition) does not bind to any HLA and/or MHC molecule (e.g. in a mammal, preferably in a human, in a non-human primate, in a sheep, in a pig, in a dog or in a rodent; or of the individual to be treated) in vivo.

According to a further preference, the compound is for intracorporeal sequestration (or intracorporeal depletion) of at least one anti-PEG antibody in an individual, preferably in the bloodstream of the individual and/or for reduction of the titre of at least one anti-PEG antibody in the individual, preferably in the bloodstream of the individual.

In an aspect, the present invention relates to a pharmaceutical composition comprising the inventive compound and at least one pharmaceutically acceptable excipient.

In embodiments, the composition is prepared for intraperitoneal, subcutaneous, intramuscular and/or intravenous administration. In particular, the composition is for repeated administration (since it is typically non-immunogenic).

In a preference, the molar ratio of PEG chains to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.

In another aspect, the compound of the present invention is for use in therapy.

In the course of the present invention, it turned out that the in vivo kinetics of undesirable-antibody lowering by the inventive compound is typically very fast, sometimes followed by a mild rebound of the undesirable antibody. It is thus particularly preferred when the compound (or the pharmaceutical composition comprising the compound) is administered at least twice within a 96-hour window, preferably within a 72-hour window, more preferably within a 48-hour window, even more preferably within a 36-hour window, yet even more preferably within a 24-hour window, especially within a 18-hour window or even within a 12-hour window; in particular wherein this window is followed by administration of the active agent as described below within 24 hours, preferably within 12 hours (but typically after at least 6 hours). For instance, the pharmaceutical composition may be administered at -24hrs and -12hrs before administration of the active agent at 0hrs.

In particular, the compound is for use in inhibiting an immune reaction of an individual to a treatment with an active agent, wherein the active agent comprises at least one PEG, in particular wherein the active agent is PEGylated. Preferably the pharmaceutical composition is administered prior to or concurrently with the active agent.

According to a further preference, the compound is for use in inhibiting neutralization, in particular accelerated blood clearance, of an active agent in an individual, wherein the active agent comprises at least one PEG, in particular wherein the active agent is PEGylated. Preferably the pharmaceutical composition is administered prior to or concurrently with the active agent.

Typically, in the context of the present invention, the active agent is a protein or peptide, preferably wherein the active agent is selected from the group of enzymes, enzyme inhibitors, antibodies, antibody fragments, antibody mimetics, antibody-drug conjugates, hormones, growth factors, clotting factors and cytokines; or the active agent may be a viral vector such as a viral vector for gene therapy or a virus-based carrier vaccination. PEGylated viral vectors are for instance disclosed in Balakrishnan et al., 2019 or Barry et al., 2020.

Most typically, in the entire context of the present invention, the active agent has a (biological) activity (e.g. an enzymatic activity and/or a therapeutic effect) which the inventive compound or biopolymer scaffold do not have.

As a further example, adenoviral vectors were shielded with PEG in order to render them less immunogenic or less susceptible to clearance by preexisting or induced antibodies (O'Riordan et al 1999), Kim et al 2012). The same concept was applied on AAV (Lee et al 2005, Weaver et al 2008. Accordingly, it is especially preferred when the active agent is a PEG-comprising adenovirus vector or carrier or a PEG-comprising AAV vector or carrier.

In particular the active agent is selected from the active agents listed in Table 1 above (as identified by the INN), pegvorhyaluronidase alfa, pegunigalsidase alfa, PEGylated arginase (e.g. BCT-100), PEGylated arginine deaminase (e.g. ADI PEG-20) and PEGylated methioninase.

PEG is also used for the modification and formulation of enzymes for the therapy of metabolic diseases and cancer, and for enzyme replacement therapies, typically for rare, inherited diseases. Enzyme replacement therapies are typically applied in hereditary metabolic conditions with absence of endogenous enzyme activities either by structural defects, low expression or absence of an enzyme that is required in a metabolic pathway. These conditions are typically associated with the accumulation of substrates or intermediates of the pathway that is affected thereby causing disease state. As an alternative, enzyme therapies can also be applied to enhance the enzymatic activity of intact enzymes of an endogenous pathway, such as listed in Table 2, below (some PEGylated enzymes are also mentioned in Table 1, above). Other applications of enzymes include cancer, neuromuscular dysfunctions, hemostasis, or even cosmetic intervention.

Examples of established PEGylated enzymes include Phenylalanine ammonia lyase (Sarkissian et al., 2008), L-asparaginase (Meneguetti 2019) or Adenosine deaminase (Booth et al. 2009), Pegunigalsidase for Fabry disease (Kant 2020) or PEGylated asparaginase enzyme Crisantaspase (Torres-Obreque 2019).

Therefore, in a further preference, the active agent is a PEGylated form of an enzyme, preferably wherein the enzyme is selected from Table 2 below.

**Table 2 Further therapeutic enzymes**

| **Enzyme** | **Therapeutic application and/or mode of action** |
|---|---|
| Acid α-glucosidase | Pompe disease |
| Acid Ceramidase | Farber disease, Cystic Fibrosis |
| Acid sphingomyelinase | Niemann-Pick Type B disease |
| α-galactosidase | Fabry disease: degradation of globotriaosylceramide |
| α-L-iduronidase | Hurler and Hurler-Scheie forms of mucopolysaccharidosis I |
| α-N-acetylglucosaminidase | Sanfilippo syndrome (MPS III B) |
| Alteplase | thrombolytic |
| Anistreplase | thrombolytic drug |
| Arylsulfatase | Maroteaux-Lamy Syndrome (MPS VI) |
| Arylsulfatase A | metachromatic leukodystrophy: cerebroside 3-sulfate |
| β-galactosidase | GM1 Gangliosidosis, Sialidosis |
| β-glucuronidase | Sly Syndrome (MPS VII) |
| β-hexosaminidase A (HexA) | GM2 Gangliosidosis, Sandhoff, Tay-Sachs disease |
| Botulinum neurotoxin type A complex | severe muscle spasticity |
| Botulism toxin type A | dystonia, spasticity |
| Botulism toxin type B | dystonia, spasticity |
| Carboxypeptidase | cancer |
| Drotrecognin-a | antithrombotic, antiinflammatory effects |
| Glucocerebrosidase | Gaucher disease: degradation of glucosylceramide |
| Hyaluronidase | hydrolysis of hyaluronic acid |
| Iduronate-2-sulfatase | Hunter Disease (MPS II) |
| L-Asparaginase | ALL, NHL |
| Lipase, amylase, protease | pancreatic insufficiency |
| Lysosomal acid lipase | lysosomal acid lipase deficiency |
| N-acetylgalactosamine 6-sulfatase | Morquio A syndrome (MPS IV A) |
| N-acetylgalactosamine-4-sulfatase | mucopolysaccharidosis VI |
| Porphobilinogen deaminase | acute intermittent porphyria |
| Rasburicase | enzymatic oxidation of uric acid into allantoin |
| Reteplase | thrombolytic |
| Streptokinase | thrombolytic |
| Tenecteplase | thrombolytic |
| Tissue-nonspecific alkaline phosphatase fusion protein | hypophosphatasia |
| Urate oxidase | treatment-refractory gout |
| Urokinase | thrombolytic |

Further, comprehensive reviews with (candidate) PEGylated drug lists in clinical and preclinical phases are provided by Park et al. (2019), Swierczewska et al. (2015) or Kang & Stevens 2009. Lysosomal Enzyme Replacement Therapies were reviewed by Solomon and Muro 2017. All enzymes mentioned in the publications cited in this paragraph (or their PEGylated form, if they are not disclosed to be PEGylated) may also be used in the present invention as active agents.

Enzymes can be combined with formulations such as PEG liposomes (see review by Solomon & Muro 2017). However, PEG liposomes can become problematic when they induce anti-PEG antibodies (Ishida 2006). The present invention is hence particularly suitable for such applications. Accordingly, PEG-comprising lipid-enzyme particles may also be used in the present invention as active agent.

In general, the main problem of gene transfer into cells and tissues is rapid clearance and degradation by nucleases and limited tissue distribution. DNAs and RNAs usually undergo rapid degradation by nucleases, rapid renal and hepatic clearance, and they are generally incapable of cellular entry owing to a combination of hydrophilicity and high molecular weight. Viral vectors are an attractive alternative as biological carriers for genetic material, however major limitations are cargo size, vector production and scale-up. Importantly, the biggest obstacle is their immunogenicity.

Therefore, non-viral gene transfer vectors composed of lipids, polymers and/or peptides in combination with nucleic acids are currently under intense investigation in different combinations. For example, polyplexes or nanocomplexes are amongst the best studied and versatile nucleic acid carriers. They protect their nucleic acid cargo from endonuclease digestion, thereby improving stability and in vivo circulation time, and they can also be used as vaccine adjuvants.

In particular, cationic nanocarriers often contain PEG to reduce unwanted cellular uptake or mucosal adjuvant effects, as seen e.g. for PEI (Polyethyleneimine) when combined with glycoprotein antigens (Wegmann 2012). Modifications of PEG structures, such as e.g. brush-, star-, or micellar PEG were developed for improving biodistribution, cellular uptake and gene expression and for reducing undesired side effects. Extensive reviews about PEGylated delivery polymers published by Suk et al. 2016, Yang et al. 2015 and Sun et al. 2019.

Despite the many general advantages of PEG for drug and vaccine delivery (in particular DNA and RNA delivery), for bioconjugation or for the development of PEG-containing formulations, such as PEG-containing or PEG-coated liposome and nanoparticle formulations (reviewed by Inglut 2020), the problems caused by PEG immunogenicity of such complexes with nucleic acids (as well as PEGylated nucleic acids themselves) have not yet been resolved in the prior art. The present invention is also suitable to address these problems.

Thus, in a particularly preferred embodiment of the present invention, the active agent is a nucleic acid-lipid particle, a nucleic acid-polymer particle (wherein the polymer may e.g. be a protein or peptide), a nucleic acid-lipid-polymer particle (wherein the polymer may e.g. be a protein or peptide), or a nucleic acid. Said nucleic acid (of the particles) may be DNA or RNA. Accordingly, the active agent may e.g. be an RNA-lipid particle, an RNA-polymer particle, an RNA-lipid-polymer particle, or an RNA. In other embodiments, the active agent may e.g. be a DNA-lipid particle, a DNA-polymer particle, a DNA-lipid-polymer particle, or a DNA. It is evident to the skilled person that the particle as used herein as active agent may for instance be a non-covalent complex of its components (i.e. nucleic acids, and lipids and/or polymers).

Importantly, also messenger ribonucleic acid (mRNA) vaccines against SARS-CoV-2, such as mRNA-1273 (Moderna Inc.) and BNT162b2 (Biontech SE/Pfizer Inc), which will be administered to hundreds of millions of individuals in the next years are based on PEG-containing lipid nanoparticles (LNPs), which are a form of nucleic acid-lipid particles, with mRNA (reviewed by Aldosari et al. 2021). SARS-Cov-2 vaccine development, including LNP-based mRNA vaccines, are reviewed for instance in Dong et al., 2020, and Kaur et. al. 2020. LNPs are for instance also disclosed in US patents US 7,404,969, US 8,058,069, US 9,364,435 and US 9,404,127. In addition, other mRNA vaccines are based on PEG-containing LNPs as well.

The clinical use of these PEG-containing-LNP-based mRNA vaccines against SARS-CoV-2 has led to severe allergic reactions in some individuals (see for instance Worm et al., 2021, which also discloses ingredients of the vaccines). Anaphylaxis associated with these vaccines is also reviewed in Turk, 2021. PEG present in the vaccines is mentioned as the most likely culprit for anaphylaxis.

Thus, in a preferred embodiment, the nucleic acid is mRNA. Accordingly, the active agent may e.g. be an mRNA-lipid particle, an mRNA-polymer particle, an mRNA-lipid-polymer particle, or an mRNA.

PEG is also used in the delivery of DNA and RNA oligonucleotides, including antisense- and splice-correcting oligonucleotides, small interfering RNAs (siRNAs) and microRNAs (miRNAs) (reviewed by Lu et al. 2019). Consequently, in a preferred embodiment, the nucleic acid (of the particles) is selected from antisense-oligonucleotides, splice-correcting oligonucleotides, small interfering RNAs (siRNAs) and microRNAs (miRNAs).

Further, PEG is used in aptamer delivery, including DNA-, RNA- and spiegelmers (reviewed by Jain et al. 2020). Therefore, in a further preferred embodiment, the nucleic acid (of the particles) is an aptamer.

In yet another preferred embodiment, the nucleic acid-polymer particle is a nucleic acid-PEG-PEI copolymer particle, as e.g. disclosed in Lutz et al. 2008, or a nucleic acid-peptide particle (e.g. wherein the nucleic acid is complexed with PEGylated cationic peptides) as e.g. disclosed in Qiu 2019.

In a further preferred embodiment, the nucleic acid-lipid particle is a cationic-lipid assisted particle (cationic lipid-assisted nanoparticle, CLAN), preferably wherein the nucleic acid is a CRISPR/Cas9 DNA plasmid, as e.g. disclosed in Luo et al 2018.

In the context of the present invention, the active agent may also be selected from any one of Tables 1-4 disclosed in Park et al. (2019) and Tables 1 and 2 of Swierczewska et al. (2015).

In embodiments, one or more anti-PEG antibodies are present in the individual.

It is highly preferred that the composition is non-immunogenic in the individual (e.g. it does not comprise an adjuvant or an immunostimulatory substance that stimulates the innate or the adaptive immune system, e.g. such as an adjuvant or a T-cell epitope).

The composition of the present invention may be administered at a dose of 1-900 mg, preferably 2-500 mg, more preferably 3-250 mg, even more preferably 4-100 mg, especially 5-50 mg, compound per kg body weight of the individual, preferably wherein the composition is administered repeatedly. Such administration may be intraperitoneally, subcutaneously, intramuscularly or intravenously.

In an aspect, the present invention relates to a method of sequestering (or depleting) one or more antibodies present in an individual, comprising
obtaining a pharmaceutical composition as defined herein, wherein the composition is non-immunogenic in the individual and wherein the one or more antibodies are anti-PEG antibodies; and
administering (in particular repeatedly administering, e.g. at least two times, preferably at least three times, more preferably at least five times) the pharmaceutical composition to the individual.

In the context of the present invention, the individual (to be treated) may be a non-human animal, preferably a non-human primate, a sheep, a pig, a dog or a rodent, in particular a mouse.

Preferably, the biopolymer scaffold is autologous with respect to the individual, preferably wherein the biopolymer scaffold is an autologous protein (i.e. murine albumin is used when the individual is a mouse).

In another aspect, the present invention relates to a pharmaceutical composition, comprising the compound of the present invention and further comprising an active agent as disclosed herein and optionally at least one pharmaceutically acceptable excipient.

This composition is preferably for use in inhibition of an immune reaction, preferably an anti-PEG-antibody-mediated immune reaction, against the active agent.

This composition is furthermore preferably non-immunogenic in the individual.

In yet another aspect, the present invention relates to a method of inhibiting a (humoral) immune reaction to a treatment with an active agent in an individual in need of treatment with the active agent, comprising obtaining a pharmaceutical composition as defined above; wherein the compound of the pharmaceutical composition is non-immunogenic in the individual, and administering (preferably repeatedly administering) the pharmaceutical composition to the individual.

In the context of the present invention, for improved bioavailability, it is preferred that the inventive compound has a solubility in water at 25°C of at least 0.1 µg/ml, preferably at least 1 µg/ml, more preferably at least 10 µg/ml, even more preferably at least 100 µg/ml, especially at least 1000 µg/ml .

The term "preventing" or "prevention" as used herein means to stop a disease state or condition from occurring in a patient or subject completely or almost completely or at least to a (preferably significant) extent, especially when the patient or subject or individual is predisposed to such a risk of contracting a disease state or condition.

The pharmaceutical composition of the present invention is preferably provided as a (typically aqueous) solution, (typically aqueous) suspension or (typically aqueous) emulsion. Excipients suitable for the pharmaceutical composition of the present invention are known to the person skilled in the art, upon having read the present specification, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives. Other suitable excipients include any compound that does not itself induce the production of antibodies in the patient (or individual) that are harmful for the patient (or individual). Examples are well tolerable proteins, polysaccharides, polylactic acids, polyglycolic acid, polymeric amino acids and amino acid copolymers. This pharmaceutical composition can (as a drug) be administered via appropriate procedures known to the skilled person (upon having read the present specification) to a patient or individual in need thereof (i.e. a patient or individual having or having the risk of developing the diseases or conditions mentioned herein). The preferred route of administration of said pharmaceutical composition is parenteral administration, in particular through intraperitoneal, subcutaneous, intramuscular and/or intravenous administration. For parenteral administration, the pharmaceutical composition of the present invention is preferably provided in injectable dosage unit form, e.g. as a solution (typically as an aqueous solution), suspension or emulsion, formulated in conjunction with the above-defined pharmaceutically acceptable excipients. The dosage and method of administration, however, depends on the individual patient or individual to be treated. Said pharmaceutical composition can be administered in any suitable dosage known from other biological dosage regimens or specifically evaluated and optimised for a given individual. For example, the active agent may be present in the pharmaceutical composition in an amount from 1 mg to 10 g, preferably 50 mg to 2 g, in particular 100 mg to 1 g. Usual dosages can also be determined on the basis of kg body weight of the patient, for example preferred dosages are in the range of 0.1 mg to 100 mg/kg body weight, especially 1 to 10 mg/kg body weight (per administration session). The administration may occur e.g. once daily, once every other day, once per week or once every two weeks. As the preferred mode of administration of the inventive pharmaceutical composition is parenteral administration, the pharmaceutical composition according to the present invention is preferably liquid or ready to be dissolved in liquid such sterile, de-ionised or distilled water or sterile isotonic phosphate-buffered saline (PBS). Preferably, 1000 µg (dry-weight) of such a composition comprises or consists of 0.1-990 µg, preferably 1-900µg, more preferably 10- 200µg compound, and option-ally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, de-ionised/distilled water or sterile isotonic phosphate-buffered saline (PBS) to yield a final volume of 0.1-100 ml, preferably 0.5-20 ml, more preferably 1-10 ml.

It is evident to the skilled person that active agents and drugs described herein can also be administered in salt-form (i.e. as a pharmaceutically acceptable salt of the active agent). Accordingly, any mention of an active agent herein shall also include any pharmaceutically acceptable salt forms thereof.

The coupling/conjugation chemistry used to link PEG chains to the biopolymer scaffold (e.g. via techniques as described in "Bioconjugate Techniques", Greg T. Hermanson) can also be selected from reactions known to the skilled in the art. The biopolymer scaffold itself may be recombinantly produced or obtained from natural sources.

Herein, the term "specific for" - as in "molecule A specific for molecule B" - means that molecule A has a binding preference for molecule B compared to other molecules in an individual's body. Typically, this entails that molecule A (such as an antibody) has a dissociation constant (also called "affinity") in regard to molecule B (such as the antigen, specifically the binding epitope thereof) that is lower than (i.e. "stronger than") 1000 nM, preferably lower than 100 nM, more preferably lower than 50 nM, even more preferably lower than 10 nM, especially lower than 5 nM.

The present invention further relates to the following embodiments:
Embodiment 1. A compound comprising
   - a biopolymer scaffold and
   - one or more PEG chains.
Embodiment 2. The compound of embodiment 1, wherein said one or more PEG chains comprise at least two, preferably at least three, more preferably at least five, even more preferably at least ten or even at least twenty PEG chains.
Embodiment 3. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 100-10000 Da.
Embodiment 4. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 200-8000 Da.
Embodiment 5. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 300-6000 Da.
Embodiment 6. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 400-5000 Da.
Embodiment 7. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 500-4000 Da.
Embodiment 8. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 600-3000 Da.
Embodiment 9. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 700-2500 Da.
Embodiment 10. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 10000 Da.
Embodiment 11. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 9000 Da.
Embodiment 12. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 8000 Da.
Embodiment 13. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 7000 Da.
Embodiment 14. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 6000 Da.
Embodiment 15. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 5000 Da.
Embodiment 16. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 4000 Da.
Embodiment 17. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 3000 Da.
Embodiment 18. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of less than 2500 Da.
Embodiment 19. The compound of embodiment 1 or 2, wherein at least one, preferably at least 10%, more preferably at least 20%, even more preferably at least 40%, yet more preferably at least 60%, yet even more preferably at least 80% or even at least 90%, especially each of the one or more PEG chains have a molecular weight of 1500-2500 Da.
Embodiment 20. The compound of any one of embodiments 1 to 19, wherein at least one, preferably at least 10%, more preferably at least 20%, even more preferably at least 40%, yet more preferably at least 60%, yet even more preferably at least 80% or even at least 90%, especially each of the one or more PEG chains have a free methoxy end group or a free hydroxyl end group.
Embodiment 21. The compound of any one of embodiments 1 to 19, wherein at least one, preferably at least 10%, more preferably at least 20%, even more preferably at least 40%, yet more preferably at least 60%, yet even more preferably at least 80% or even at least 90%, especially each of the one or more PEG chains have a free methoxy end group.
Embodiment 22. The compound of any one of embodiments 1 to 19, wherein at least one, preferably at least 10%, more preferably at least 20%, even more preferably at least 40%, yet more preferably at least 60%, yet even more preferably at least 80% or even at least 90%, especially each of the one or more PEG chains have a free hydroxyl end group.
Embodiment 23. The compound of any one of embodiments 1 to 22, wherein the biopolymer scaffold is a protein, preferably a mammalian protein such as a human protein, a non-human primate protein, a sheep protein, a pig protein, a dog protein or a rodent protein.
Embodiment 24. The compound of embodiment 23, wherein the biopolymer scaffold is a globulin.
Embodiment 25. The compound of embodiment 24, wherein the biopolymer scaffold is selected from the group consisting of immunoglobulins, alpha1-globulins, alpha2-globulins and beta-globulins.
Embodiment 26. The compound of embodiment 25, wherein the biopolymer scaffold is selected from the group consisting of immunoglobulin G, haptoglobin and transferrin.
Embodiment 27. The compound of embodiment 26, wherein the biopolymer scaffold is transferrin.
Embodiment 28. The compound of embodiment 23, wherein the biopolymer scaffold is an albumin.
Embodiment 29. The compound of embodiment 23, wherein the biopolymer scaffold is an anti-CD163 antibody (i.e. an antibody specific for a CD163 protein) or CD163-binding fragment thereof.
Embodiment 30. The compound of embodiment 29, wherein the anti-CD163 antibody or CD163-binding fragment thereof is specific for human CD163 and/or is specific for the extracellular region of CD163, preferably for an SRCR domain of CD163, more preferably for any one of SRCR domains 1-9 of CD163, even more preferably for any one of SRCR domains 1-3 of CD163, especially for SRCR domain 1 of CD163.
Embodiment 31. The compound of embodiment 29, wherein the anti-CD163 antibody or CD163-binding fragment thereof is specific for one of the following peptides:
   a peptide consisting of 7-25, preferably 8-20, even more preferably 9-15, especially 10-13 amino acids, wherein the peptide comprises the amino acid sequence CSGRVEVKVQEEWGTVCNNGWSMEA (SEQ ID NO: 3) or a 7-24 amino-acid fragment thereof,
   a peptide consisting of 7-25, preferably 8-20, even more preferably 9-15, especially 10-13 amino acids, wherein the peptide comprises the amino acid sequence DHVSCRGNESALWDCKHDGWG (SEQ ID NO: 13) or a 7-20 amino-acid fragment thereof, or
   a peptide consisting of 7-25, preferably 8-20, even more preferably 9-15, especially 10-13 amino acids, wherein the peptide comprises the amino acid sequence SSLGGTDKELRLVDGENKCS (SEQ ID NO: 24) or a 7-19 amino-acid fragment thereof.
Embodiment 32. The compound of embodiment 31, wherein the anti-CD163 antibody or CD163-binding fragment thereof is specific for a peptide comprising the amino acid sequence ESALW (SEQ ID NO: 14) or ALW.
Embodiment 33. The compound of embodiment 31, wherein the anti-CD163 antibody or CD163-binding fragment thereof is specific for a peptide comprising the amino acid sequence GRVEVKVQEEW (SEQ ID NO: 4), WGTVCNNGWS (SEQ ID NO: 5) or WGTVCNNGW (SEQ ID NO: 6).
Embodiment 34. The compound of embodiment 31, wherein the anti-CD163 antibody or CD163-binding fragment thereof is specific for a peptide comprising the amino acid sequence SSLGGTDKELR (SEQ ID NO: 25) or SSLGG (SEQ ID NO: 26).
Embodiment 35. The compound of any one of embodiments 1 to 34, wherein the compound is non-immunogenic in a mammal, preferably in a human, in a non-human primate, in a sheep, in a pig, in a dog or in a rodent.
Embodiment 36. The compound of any one of embodiments 1 to 35, wherein the compound is for intracorporeal sequestration (or intracorporeal depletion or intracorporeal blocking) of at least one anti-PEG antibody in an individual, preferably in the bloodstream of the individual and/or for reduction of the titre of at least one anti-PEG antibody in the individual, preferably in the bloodstream of the individual.
Embodiment 37. The compound of any one of embodiments 1 to 36, wherein each of the one or more PEG chains is covalently bound to the biopolymer scaffold, preferably via a linker each.
Embodiment 38. The compound of any one of embodiments 1 to 36, wherein at least a portion of the one or more PEG chains are covalently bound to the biopolymer scaffold via at least one linker.
Embodiment 39. The compound of embodiment 37 or 38, wherein the linker comprises a peptide or a single amino acid such as a cysteine.
Embodiment 40. The compound of embodiment 39, wherein the linker comprises the peptide.
Embodiment 41. The compound of embodiment 40, wherein the peptide has a sequence length of 2-13 amino acids, preferably 3-11 amino acids, more preferably 4-9 amino acids, especially 5-8 amino acids.
Embodiment 42. The compound of embodiments 40 or 41, wherein the peptide is linear or circularized.
Embodiment 43. The compound of any one of embodiments 40 to 42, wherein the peptide is non-immunogenic in a mammal, preferably in a human, in a non-human primate, in a sheep, in a pig, in a dog or in a rodent.
Embodiment 44. The compound of any one of embodiments 40 to 43, wherein the peptide contains at least one lysine residue, preferably at least two, more preferably at least three, even more preferably at least four, especially at least five lysine residues; or wherein the peptide contains at least one tyrosine residue, preferably at least two, more preferably at least three, even more preferably at least four, especially at least five tyrosine residues; or wherein the peptide contains at least one cysteine residue, preferably at least two, more preferably at least three, even more preferably at least four, especially at least five cysteine residues.
Embodiment 45. The compound of any one of embodiments 40 to 44, wherein the peptide contains at least one glycine residue, preferably at least two, more preferably at least three, even more preferably at least four, especially at least five glycine residues.
Embodiment 46. The compound of any one of embodiments 40 to 45, wherein the peptide has a terminal cysteine residue.
Embodiment 47. The compound of any one of embodiments 40 to 46, wherein the peptide comprises the amino-acid sequence (X₁-(X₂)ₘ)ₙ, wherein m is an integer from 1 to 5, preferably from 2 to 4, wherein n is an integer from 1 to 5, preferably from 2 to 5;
Embodiment 48. The compound of embodiment 47, wherein, independently for each occurrence, X₁ is lysine, tyrosine, tryptophan, methionine, histidine or cysteine, and, independently for each occurrence, X₂ is any other amino acid that is not X₁, preferably glycine.
Embodiment 49. The compound of any one of embodiments 38 to 48, wherein said portion comprises at least 10%, preferably at least 20%, more preferably at least 30%, even more preferably at least 40%, yet even more preferably at least 50% or even 75%, especially all of the one or more PEG chains.
Embodiment 50. The compound of any one of embodiments 38 to 49, wherein at least two, preferably at least three, more preferably at least four, especially at least five PEG chains are bound to a single of the at least one linker, preferably to the peptide of the linker.
Embodiment 51. The compound of any one of embodiments 1 to 50, wherein the biopolymer scaffold is human transferrin.
Embodiment 52. The compound of any one of embodiments 1 to 51, wherein the compound is non-immunogenic in humans.
Embodiment 53. A pharmaceutical composition comprising the compound of any one of embodiments 1 to 52 and at least one pharmaceutically acceptable excipient.
Embodiment 54. The pharmaceutical composition of embodiment 53, wherein the composition is prepared for intraperitoneal, subcutaneous, intramuscular and/or intravenous administration and/or wherein the composition is for repeated administration.
Embodiment 55. The pharmaceutical composition of embodiment 53 or 54, wherein the molar ratio of PEG chains to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.
Embodiment 56. The pharmaceutical composition of any one of embodiments 53 to 55 for use in therapy.
Embodiment 57. The pharmaceutical composition for use according to embodiment 56, for use in inhibiting an immune reaction of an individual to a treatment with an active agent, wherein the active agent comprises at least one PEG, in particular wherein the active agent is PEGylated; preferably wherein the pharmaceutical composition is administered prior to or concurrently with the active agent, more preferably wherein the pharmaceutical composition is administered at least twice within a 96-hour window, preferably within a 72-hour window, more preferably within a 48-hour window, even more preferably within a 36-hour window, yet even more preferably within a 24-hour window, especially within a 18-hour window or even within a 12-hour window, especially wherein this window is followed by administration of the active agent within 24 hours, preferably within 12 hours.
Embodiment 58. The pharmaceutical composition for use according to embodiment 56, for use in inhibiting neutralization, in particular accelerated blood clearance, of an active agent in an individual, wherein the active agent comprises at least one PEG, in particular wherein the active agent is PEGylated; preferably wherein the pharmaceutical composition is administered prior to or concurrently with the active agent, more preferably wherein the pharmaceutical composition is administered at least twice within a 96-hour window, preferably within a 72-hour window, more preferably within a 48-hour window, even more preferably within a 36-hour window, yet even more preferably within a 24-hour window, especially within a 18-hour window or even within a 12-hour window, especially wherein this window is followed by administration of the active agent within 24 hours, preferably within 12 hours.
Embodiment 59. The pharmaceutical composition for use according to embodiments 57 or 58, wherein the active agent is a protein or peptide, preferably wherein the active agent is selected from the group of enzymes, enzyme inhibitors, antibodies, antibody fragments, antibody mimetics, antibody-drug conjugates, hormones, growth factors, clotting factors and cytokines; or wherein the active agent is a nucleic acid-lipid particle, a nucleic acid-polymer particle, a nucleic acid-lipid-polymer particle, or a nucleic acid, or wherein the active agent is a viral vector such as a viral vector for gene therapy or vaccination; in particular wherein the active agent is selected from the active agents listed in Table 1 (as identified by the INN), pegvorhyaluronidase alfa, pegunigalsidase alfa, PEGylated arginase (e.g. BCT-100), PEGylated arginine deaminase (e.g. ADI PEG-20), PEGylated methioninase and PEGylated forms of enzymes listed in Table 2.
Embodiment 60. The pharmaceutical composition for use according to any one of embodiments 57 to 59, wherein one or more anti-PEG antibodies are present in the individual.
Embodiment 61. The pharmaceutical composition for use according to any one of embodiments 57 to 60, wherein the composition is non-immunogenic in the individual.
Embodiment 62. The pharmaceutical composition for use according to any one of embodiments 57 to 61, wherein the composition is administered at a dose of 1-900 mg, preferably 2-500 mg, more preferably 3-250 mg, even more preferably 4-100 mg, especially 5-50 mg, compound per kg body weight of the individual.
Embodiment 63. The pharmaceutical composition for use according to any one of embodiments 57 to 62, wherein the composition is administered intraperitoneally, subcutaneously, intramuscularly or intravenously.
Embodiment 64. A method of sequestering (or depleting) one or more antibodies present in an individual, comprising
   obtaining a pharmaceutical composition as defined in any one of embodiments 53 to 63, wherein the composition is non-immunogenic in the individual and wherein the one or more antibodies are anti-PEG antibodies; and
   administering the pharmaceutical composition to the individual;
   preferably wherein said administering is prior to or concurrently with administration of an active agent such as a viral vector or a protein or peptide, especially selected from the group of enzymes, enzyme inhibitors, antibodies, antibody fragments, antibody mimetics, antibody-drug conjugates, hormones, growth factors, clotting factors and cytokines (in particular wherein the active agent is selected from the active agents listed in Table 1 as identified by the INN, pegvorhyaluronidase alfa, pegunigalsidase alfa, PEGylated arginase (e.g. BCT-100), PEGylated arginine deaminase (e.g. ADI PEG-20), PEGylated methioninase and PEGylated forms of enzymes listed in Table 2), or such as a nucleic acid-lipid particle, a nucleic acid-polymer particle, a nucleic acid-lipid-polymer particle, or a nucleic acid, and wherein the active agent comprises at least one PEG, in particular wherein the active agent is PEGylated.
Embodiment 65. The method of embodiment 64, wherein the individual is a non-human animal, preferably a non-human primate, a sheep, a pig, a dog or a rodent, in particular a mouse.
Embodiment 66. The method of embodiments 64 or 65, wherein the biopolymer scaffold is autologous with respect to the individual, preferably wherein the biopolymer scaffold is an autologous protein.
Embodiment 67. The method of any one of embodiments 64 to 66, wherein the composition is administered intraperitoneally, subcutaneously, intramuscularly or intravenously.
Embodiment 68. A pharmaceutical composition, comprising the compound of any one of embodiments 1 to 52 and further comprising an active agent, wherein the active agent comprises at least one PEG, in particular wherein the active agent is PEGylated.
Embodiment 69. The pharmaceutical composition of embodiment 68, wherein the active agent is a nucleic acid-lipid particle, a nucleic acid-polymer particle, a nucleic acid-lipid-polymer particle, or a nucleic acid, or a viral vector or a protein or peptide, especially selected from the group of enzymes, enzyme inhibitors, antibodies, antibody fragments, antibody mimetics, antibody-drug conjugates, hormones, growth factors, clotting factors and cytokines; in particular wherein the active agent is selected from the active agents listed in Table 1 (as identified by the INN), pegvorhyaluronidase alfa, pegunigalsidase alfa, PEGylated arginase (e.g. BCT-100), PEGylated arginine deaminase (e.g. ADI PEG-20), PEGylated methioninase and PEGylated forms of enzymes listed in Table 2.
Embodiment 70. The pharmaceutical composition of any one of embodiments 68 to 69, wherein the composition is prepared for intravenous administration.
Embodiment 71. The pharmaceutical composition of any one of embodiments 68 to 71, wherein the composition is an aqueous solution.
Embodiment 72. The pharmaceutical composition of any one of embodiments 68 to 71 for use in inhibition of an immune reaction, preferably an anti-PEG-antibody-mediated immune reaction, against the active agent.
Embodiment 73. The pharmaceutical composition for use according to embodiment 72, wherein the composition is non-immunogenic in the individual.
Embodiment 74. A method of inhibiting an immune reaction to a treatment with an active agent in an individual in need of treatment with the active agent, comprising
   obtaining a pharmaceutical composition as defined in any one of embodiments 68 to 74; wherein the compound of the pharmaceutical composition is non-immunogenic in the individual, and
   administering the pharmaceutical composition to the individual.
Embodiment 75. The method of embodiment 74, wherein the individual is human.
Embodiment 76. The method of embodiment 74 or 75, wherein the biopolymer scaffold is autologous with respect to the individual, preferably wherein the biopolymer scaffold is an autologous protein.
Embodiment 77. The method of any one of embodiments 74 to 76, wherein the composition is administered intraperitoneally, subcutaneously, intramuscularly or intravenously.

Due to pre-existing and induced anti-PEG antibodies in a considerable portion of patients, alternative protein modifications were suggested in the art, to reduce immunogenicity and increase stability of biological drugs and to avoid accelerated blood clearance. In particular, it was suggested to modify active agents with disordered and hydrophilic polypeptides (i.e. by XTENylation or PASylation), by polysialylation, or by methylation (in particular reductive methylation of lysines which can increase cytosolic stability of protein drugs). Furthermore, the attachment of carbohydrates to proteins was suggested (glycosylation). Although generally regarded as beneficial when shielding a protein drug from the immune system is required, many of these modifications are nevertheless recognized as foreign by the immune system, and have the potential to bind to pre-existing antibodies or to induce specific antibodies (reviewed by Zinsli 2020).

XTENylation is for instance described in detail in Podust et al as well as in WO 2013/130683 A2. As used herein, XTENylation is understood to be a modification with a polypeptide, the polypeptide having the following features:
a. the polypeptide comprises 36 to 3000 amino acid residues; and
b. the sum of glycine (G), alanine (A), serine (S), threonine (T), glutamate (E) and proline (P) residues constitutes more than about 90% of the total amino acid residues of the polypeptide.

Furthermore, the polypeptide has preferably at least one, more preferably at least two, even more preferably at least three, especially all of features c-f (see also WO 2013/130683 A2):
c. the polypeptide sequence is substantially non-repetitive such that (i) the polypeptide sequence contains no three contiguous amino acids that are identical unless the amino acids are serine, and (ii) at least 80% of the polypeptide sequence consists of non-overlapping sequence motifs, each of the sequence motifs comprising at least 9 to 14 amino acid residues and wherein each of the motifs consists of 4 to 6 types of amino acids selected from glycine (G), alanine (A), serine (S), threonine (T), glutamate (E) and proline (P), wherein any two contiguous amino acid residues do not occur more than twice in each of the sequence motifs;
d. the polypeptide sequence has greater than 90% random coil formation as determined by GOR algorithm;
e. the polypeptide sequence has less than 2% alpha helices and 2% beta-sheets as determined by Chou-Fasman algorithm; and
f. the polypeptide sequence lacks a predicted T-cell epitope when analyzed by TEPITOPE algorithm, wherein the TEPITOPE algorithm prediction for epitopes within the XTEN sequence is based on a score of -6.

The GOR algorithm, the Chou-Fasman algorithm and the TEPITOPE algorithm are preferably applied as disclosed in WO 2013/130683 A2.

PASylation is for instance described in detail in Binder and Skerra, 2017, as well as in WO 2008/155134 A1 and WO 2011/144756 A1. As used herein, PASylation is understood to be a modification with a polypeptide, the polypeptide comprising an amino acid sequence of at least 50, preferably at least 100, amino acid residues forming a random coil conformation. In particular, this amino acid sequence consists of alanine, proline and, optionally, serine residues. In this context, the term "random coil" shall be understood as defined in WO 2008/155134 A1.

It turned out that the inventive concept developed originally for PEG-contained active agents can also be applied to these other modifications. Inventive compounds which carry these above-mentioned moieties are highly suitable to remove antibodies that are causing accelerated blood clearance of modified active agents, in particular biologics shielded by these moieties, by binding to these above-mentioned shielding moieties.

The present invention thus further relates to the following embodiments:
Embodiment A1. A compound comprising
   - a biopolymer scaffold and
   - one or more modifications selected from the group of PEGylation, XTENylation, PASylation, methylation, glycosylation, and polysialylation.
Embodiment A2. The compound of embodiment A1, wherein said one or more modifications comprise at least two, preferably at least three, more preferably at least five, even more preferably at least ten or even at least twenty modifications.
Embodiment A3. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 100-10000 Da.
Embodiment A4. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 200-8000 Da.
Embodiment A5. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 300-6000 Da.
Embodiment A6. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 400-5000 Da.
Embodiment A7. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 500-4000 Da.
Embodiment A8. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 600-3000 Da.
Embodiment A9. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 700-2500 Da.
Embodiment A10. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 10000 Da.
Embodiment A11. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 9000 Da.
Embodiment A12. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 8000 Da.
Embodiment A13. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 7000 Da.
Embodiment A14. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 6000 Da.
Embodiment A15. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 5000 Da.
Embodiment A16. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 4000 Da.
Embodiment A17. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 3000 Da.
Embodiment A18. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of less than 2500 Da.
Embodiment A19. The compound of embodiment A1 or A2, wherein at least one, preferably at least 10%, more preferably at least 20%, even more preferably at least 40%, yet more preferably at least 60%, yet even more preferably at least 80% or even at least 90%, especially each of the one or more modifications have a molecular weight of 1500-2500 Da.
Embodiment A20. The compound of any one of embodiments A1 to A19, wherein at least one, preferably at least 10%, more preferably at least 20%, even more preferably at least 40%, yet more preferably at least 60%, yet even more preferably at least 80% or even at least 90%, especially each of the one or more modifications have a free end group such as a free methoxy end group or a free hydroxyl end group.
Embodiment A21. The compound of any one of embodiments A1 to A19, wherein at least one, preferably at least 10%, more preferably at least 20%, even more preferably at least 40%, yet more preferably at least 60%, yet even more preferably at least 80% or even at least 90%, especially each of the one or more modifications have a free amino end group or a free carboxyl end group.
Embodiment A22. The compound of any one of embodiments A1 to A19, wherein at least one, preferably at least 10%, more preferably at least 20%, even more preferably at least 40%, yet more preferably at least 60%, yet even more preferably at least 80% or even at least 90%, especially each of the one or more modifications have a free carbohydrate end group or a free sialic acid end group.
Embodiment A23. The compound of any one of embodiments A1 to A22, wherein the biopolymer scaffold is a protein, preferably a mammalian protein such as a human protein, a non-human primate protein, a sheep protein, a pig protein, a dog protein or a rodent protein.
Embodiment A24. The compound of embodiment A23, wherein the biopolymer scaffold is a globulin.
Embodiment A25. The compound of embodiment A24, wherein the biopolymer scaffold is selected from the group consisting of immunoglobulins, alpha1-globulins, alpha2-globulins and beta-globulins.
Embodiment A26. The compound of embodiment A25, wherein the biopolymer scaffold is selected from the group consisting of immunoglobulin G, haptoglobin and transferrin.
Embodiment A27. The compound of embodiment A26, wherein the biopolymer scaffold is transferrin.
Embodiment A28. The compound of embodiment A23, wherein the biopolymer scaffold is an albumin.
Embodiment A29. The compound of embodiment A23, wherein the biopolymer scaffold is an anti-CD163 antibody (i.e. an antibody specific for a CD163 protein) or CD163-binding fragment thereof.
Embodiment A30. The compound of embodiment A29, wherein the anti-CD163 antibody or CD163-binding fragment thereof is specific for human CD163 and/or is specific for the extracellular region of CD163, preferably for an SRCR domain of CD163, more preferably for any one of SRCR domains 1-9 of CD163, even more preferably for any one of SRCR domains 1-3 of CD163, especially for SRCR domain 1 of CD163.
Embodiment A31. The compound of embodiment A29, wherein the anti-CD163 antibody or CD163-binding fragment thereof is specific for one of the following peptides:
   a peptide consisting of 7-25, preferably 8-20, even more preferably 9-15, especially 10-13 amino acids, wherein the peptide comprises the amino acid sequence CSGRVEVKVQEEWGTVCNNGWSMEA (SEQ ID NO: 3) or a 7-24 amino-acid fragment thereof,
   a peptide consisting of 7-25, preferably 8-20, even more preferably 9-15, especially 10-13 amino acids, wherein the peptide comprises the amino acid sequence DHVSCRGNESALWDCKHDGWG (SEQ ID NO: 13) or a 7-20 amino-acid fragment thereof, or
   a peptide consisting of 7-25, preferably 8-20, even more preferably 9-15, especially 10-13 amino acids, wherein the peptide comprises the amino acid sequence SSLGGTDKELRLVDGENKCS (SEQ ID NO: 24) or a 7-19 amino-acid fragment thereof.
Embodiment A32. The compound of embodiment A31, wherein the anti-CD163 antibody or CD163-binding fragment thereof is specific for a peptide comprising the amino acid sequence ESALW (SEQ ID NO: 14) or ALW.
Embodiment A33. The compound of embodiment A31, wherein the anti-CD163 antibody or CD163-binding fragment thereof is specific for a peptide comprising the amino acid sequence GRVEVKVQEEW (SEQ ID NO: 4), WGTVCNNGWS (SEQ ID NO: 5) or WGTVCNNGW (SEQ ID NO: 6).
Embodiment A34. The compound of embodiment A31, wherein the anti-CD163 antibody or CD163-binding fragment thereof is specific for a peptide comprising the amino acid sequence SSLGGTDKELR (SEQ ID NO: 25) or SSLGG (SEQ ID NO: 26).
Embodiment A35. The compound of any one of embodiments A1 to A34, wherein the compound is non-immunogenic in a mammal, preferably in a human, in a non-human primate, in a sheep, in a pig, in a dog or in a rodent.
Embodiment A36. The compound of any one of embodiments A1 to A35, wherein the compound is for intracorporeal sequestration (or intracorporeal depletion or intracorporeal blocking) of at least one antibody specific for the one or more modifications in an individual, preferably in the bloodstream of the individual and/or for reduction of the titre of at least one antibody specific for the one or more modifications in the individual, preferably in the bloodstream of the individual.
Embodiment A37. The compound of any one of embodiments A1 to A36, wherein each of the one or more modifications is covalently bound to the biopolymer scaffold, preferably via a linker each.
Embodiment A38. The compound of any one of embodiments A1 to A36, wherein at least a portion of the one or more modifications are covalently bound to the biopolymer scaffold via at least one linker.
Embodiment A39. The compound of embodiment A37 or A38, wherein the linker comprises a peptide or a single amino acid such as a cysteine.
Embodiment A40. The compound of embodiment A39, wherein the linker comprises the peptide.
Embodiment A41. The compound of embodiment A40, wherein the peptide has a sequence length of 2-13 amino acids, preferably 3-11 amino acids, more preferably 4-9 amino acids, especially 5-8 amino acids.
Embodiment A42. The compound of embodiments A40 or A41, wherein the peptide is linear or circularized.
Embodiment A43. The compound of any one of embodiments A40 to A42, wherein the peptide is non-immunogenic in a mammal, preferably in a human, in a non-human primate, in a sheep, in a pig, in a dog or in a rodent.
Embodiment A44. The compound of any one of embodiments A40 to A43, wherein the peptide contains at least one lysine residue, preferably at least two, more preferably at least three, even more preferably at least four, especially at least five lysine residues; or wherein the peptide contains at least one tyrosine residue, preferably at least two, more preferably at least three, even more preferably at least four, especially at least five tyrosine residues; or wherein the peptide contains at least one cysteine residue, preferably at least two, more preferably at least three, even more preferably at least four, especially at least five cysteine residues.
Embodiment A45. The compound of any one of embodiments A40 to A44, wherein the peptide contains at least one glycine residue, preferably at least two, more preferably at least three, even more preferably at least four, especially at least five glycine residues.
Embodiment A46. The compound of any one of embodiments A40 to A45, wherein the peptide has a terminal cysteine residue.
Embodiment A47. The compound of any one of embodiments A40 to A46, wherein the peptide comprises the amino-acid sequence (X₁-(X₂)ₘ)ₙ, wherein m is an integer from 1 to 5, preferably from 2 to 4, wherein n is an integer from 1 to 5, preferably from 2 to 5;
Embodiment A48. The compound of embodiment A47, wherein, independently for each occurrence, X₁ is lysine, tyrosine, tryptophan, methionine, histidine or cysteine, and, independently for each occurrence, X₂ is any other amino acid that is not X₁, preferably glycine.
Embodiment A49. The compound of any one of embodiments A38 to A48, wherein said portion comprises at least 10%, preferably at least 20%, more preferably at least 30%, even more preferably at least 40%, yet even more preferably at least 50% or even 75%, especially all of the one or more modifications.
Embodiment A50. The compound of any one of embodiments A38 to A49, wherein at least two, preferably at least three, more preferably at least four, especially at least five modifications are bound to a single of the at least one linker, preferably to the peptide of the linker.
Embodiment A51. The compound of any one of embodiments A1 to A50, wherein the biopolymer scaffold is human transferrin.
Embodiment A52. The compound of any one of embodiments A1 to A51, wherein the compound is non-immunogenic in humans.
Embodiment A53. A pharmaceutical composition comprising the compound of any one of embodiments A1 to A52 and at least one pharmaceutically acceptable excipient.
Embodiment A54. The pharmaceutical composition of embodiment A53, wherein the composition is prepared for intraperitoneal, subcutaneous, intramuscular and/or intravenous administration and/or wherein the composition is for repeated administration.
Embodiment A55. The pharmaceutical composition of embodiment A53 or A54, wherein the molar ratio of modifications to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.
Embodiment A56. The pharmaceutical composition of any one of embodiments A53 to A55 for use in therapy.
Embodiment A57. The pharmaceutical composition for use according to embodiment A56, for use in inhibiting an immune reaction of an individual to a treatment with an active agent, wherein the active agent comprises at least one of the one or more modifications; preferably wherein the pharmaceutical composition is administered prior to or concurrently with the active agent, more preferably wherein the pharmaceutical composition is administered at least twice within a 96-hour window, preferably within a 72-hour window, more preferably within a 48-hour window, even more preferably within a 36-hour window, yet even more preferably within a 24-hour window, especially within a 18-hour window or even within a 12-hour window, especially wherein this window is followed by administration of the active agent within 24 hours, preferably within 12 hours.
Embodiment A58. The pharmaceutical composition for use according to embodiment A56, for use in inhibiting neutralization, in particular accelerated blood clearance, of an active agent in an individual, wherein the active agent comprises at least one of the one or more modifications; preferably wherein the pharmaceutical composition is administered prior to or concurrently with the active agent, more preferably wherein the pharmaceutical composition is administered at least twice within a 96-hour window, preferably within a 72-hour window, more preferably within a 48-hour window, even more preferably within a 36-hour window, yet even more preferably within a 24-hour window, especially within a 18-hour window or even within a 12-hour window, especially wherein this window is followed by administration of the active agent within 24 hours, preferably within 12 hours.
Embodiment A59. The pharmaceutical composition for use according to embodiments A57 or A58, wherein the active agent is a protein or peptide, preferably wherein the active agent is selected from the group of enzymes, enzyme inhibitors, antibodies, antibody fragments, antibody mimetics, antibody-drug conjugates, hormones, growth factors, clotting factors and cytokines; or wherein the active agent is a nucleic acid-lipid particle, a nucleic acid-polymer particle, a nucleic acid-lipid-polymer particle, or a nucleic acid, or wherein the active agent is a viral vector such as a viral vector for gene therapy or vaccination; in particular wherein the active agent is selected from the active agents listed in Table 1 (as identified by the INN), pegvorhyaluronidase alfa, pegunigalsidase alfa, PEGylated arginase (e.g. BCT-100), PEGylated arginine deaminase (e.g. ADI PEG-20), PEGylated methioninase and forms of enzymes listed in Table 2 having the one or more modifications.
Embodiment A60. The pharmaceutical composition for use according to any one of embodiments A57 to A59, wherein one or more antibodies specific for the one or more modifications are present in the individual.
Embodiment A61. The pharmaceutical composition for use according to any one of embodiments A57 to A60, wherein the composition is non-immunogenic in the individual.
Embodiment A62. The pharmaceutical composition for use according to any one of embodiments A57 to A61, wherein the composition is administered at a dose of 1-900 mg, preferably 2-500 mg, more preferably 3-250 mg, even more preferably 4-100 mg, especially 5-50 mg, compound per kg body weight of the individual.
Embodiment A63. The pharmaceutical composition for use according to any one of embodiments A57 to A62, wherein the composition is administered intraperitoneally, subcutaneously, intramuscularly or intravenously.
Embodiment A64. A method of sequestering (or depleting) one or more antibodies present in an individual, comprising
   obtaining a pharmaceutical composition as defined in any one of embodiments A53 to A63, wherein the composition is non-immunogenic in the individual and wherein the one or more antibodies are antibodies specific for the one or more modifications; and
   administering the pharmaceutical composition to the individual;
   preferably wherein said administering is prior to or concurrently with administration of an active agent such as a viral vector or a protein or peptide, especially selected from the group of enzymes, enzyme inhibitors, antibodies, antibody fragments, antibody mimetics, antibody-drug conjugates, hormones, growth factors, clotting factors and cytokines (in particular wherein the active agent is selected from the active agents listed in Table 1 as identified by the INN, pegvorhyaluronidase alfa, pegunigalsidase alfa, PEGylated arginase (e.g. BCT-100), PEGylated arginine deaminase (e.g. ADI PEG-20), PEGylated methioninase and forms of enzymes listed in Table 2 having the one or more modifications), or such as a nucleic acid-lipid particle, a nucleic acid-polymer particle, a nucleic acid-lipid-polymer particle, or a nucleic acid, and wherein the active agent comprises at least one of the one or more modifications.
Embodiment A65. The method of embodiment A64, wherein the individual is a non-human animal, preferably a non-human primate, a sheep, a pig, a dog or a rodent, in particular a mouse.
Embodiment A66. The method of embodiments A64 or A65, wherein the biopolymer scaffold is autologous with respect to the individual, preferably wherein the biopolymer scaffold is an autologous protein.
Embodiment A67. The method of any one of embodiments A64 to A66, wherein the composition is administered intraperitoneally, subcutaneously, intramuscularly or intravenously.
Embodiment A68. A pharmaceutical composition, comprising the compound of any one of embodiments A1 to A52 and further comprising an active agent, wherein the active agent comprises at least one of the one or more modifications.
Embodiment A69. The pharmaceutical composition of embodiment A68, wherein the active agent is a nucleic acid-lipid particle, a nucleic acid-polymer particle, a nucleic acid-lipid-polymer particle, or a nucleic acid, or a viral vector or a protein or peptide, especially selected from the group of enzymes, enzyme inhibitors, antibodies, antibody fragments, antibody mimetics, antibody-drug conjugates, hormones, growth factors, clotting factors and cytokines; in particular wherein the active agent is selected from the active agents listed in Table 1 (as identified by the INN), pegvorhyaluronidase alfa, pegunigalsidase alfa, PEGylated arginase (e.g. BCT-100), PEGylated arginine deaminase (e.g. ADI PEG-20), PEGylated methioninase and forms of enzymes listed in Table 2 having the one or more modifications.
Embodiment A70. The pharmaceutical composition of any one of embodiments A68 to A69, wherein the composition is prepared for intravenous administration.
Embodiment A71. The pharmaceutical composition of any one of embodiments A68 to A71, wherein the composition is an aqueous solution.
Embodiment A72. The pharmaceutical composition of any one of embodiments A68 to A71 for use in inhibition of an immune reaction, preferably an immune reaction mediated by an antibody specific for the one or more modifications, against the active agent.
Embodiment A73. The pharmaceutical composition for use according to embodiment A72, wherein the composition is non-immunogenic in the individual.
Embodiment A74. A method of inhibiting an immune reaction to a treatment with an active agent in an individual in need of treatment with the active agent, comprising
   obtaining a pharmaceutical composition as defined in any one of embodiments A68 to A74; wherein the compound of the pharmaceutical composition is non-immunogenic in the individual, and
   administering the pharmaceutical composition to the individual.
Embodiment A75. The method of embodiment A74, wherein the individual is human.
Embodiment A76. The method of embodiment A74 or A75, wherein the biopolymer scaffold is autologous with respect to the individual, preferably wherein the biopolymer scaffold is an autologous protein.
Embodiment A77. The method of any one of embodiments A74 to A76, wherein the composition is administered intraperitoneally, subcutaneously, intramuscularly or intravenously.

The present invention is further illustrated by the following figures and examples, without being restricted thereto. In the context of the following figures and examples the compound on which the inventive approach is based is also referred to as "Selective Antibody Depletion Compound" (SADC).

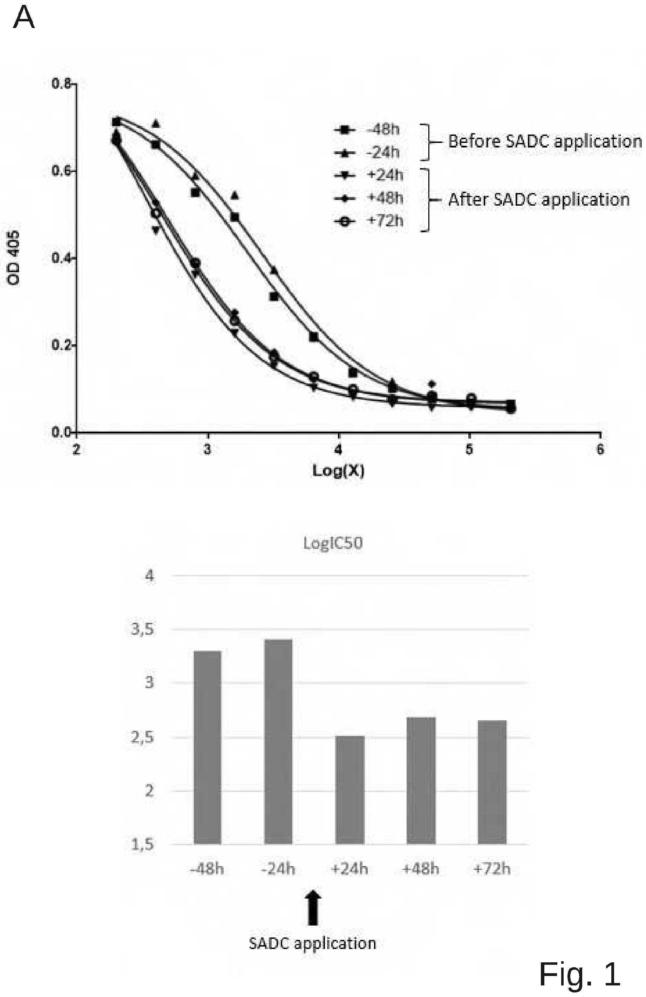

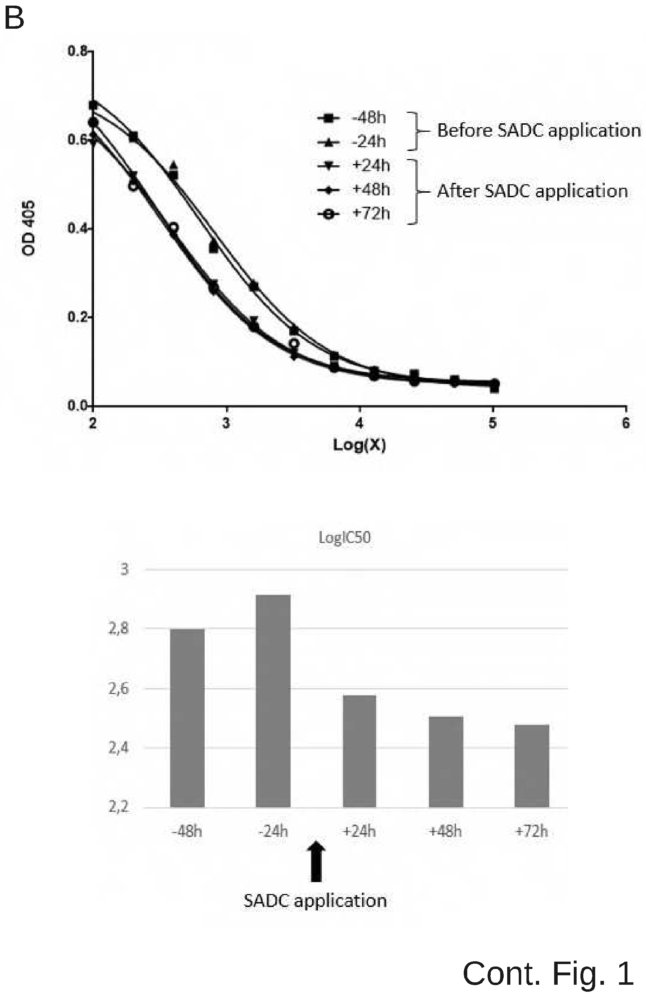

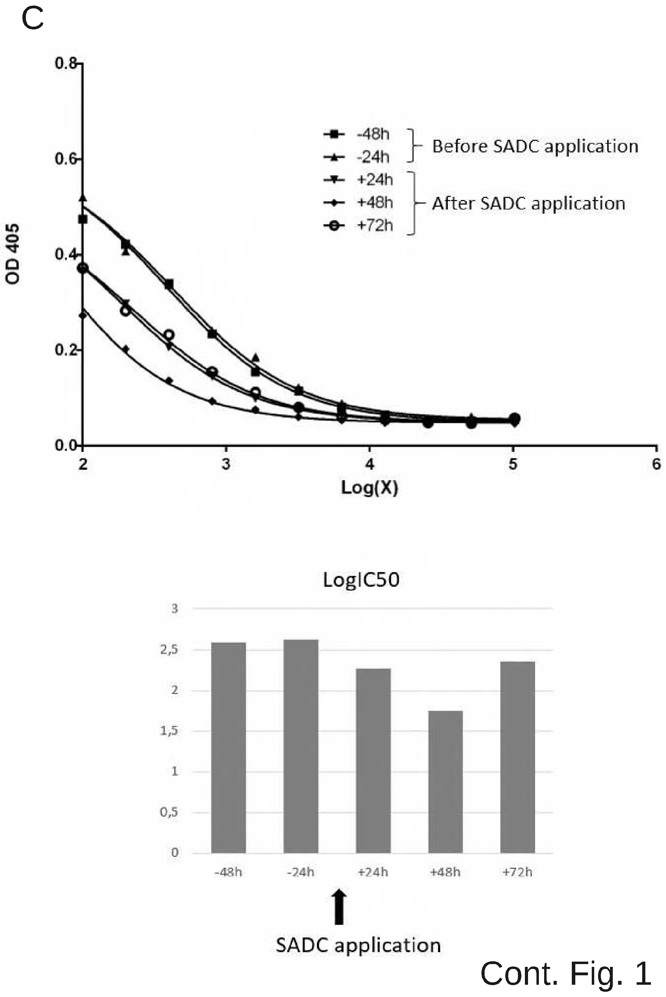

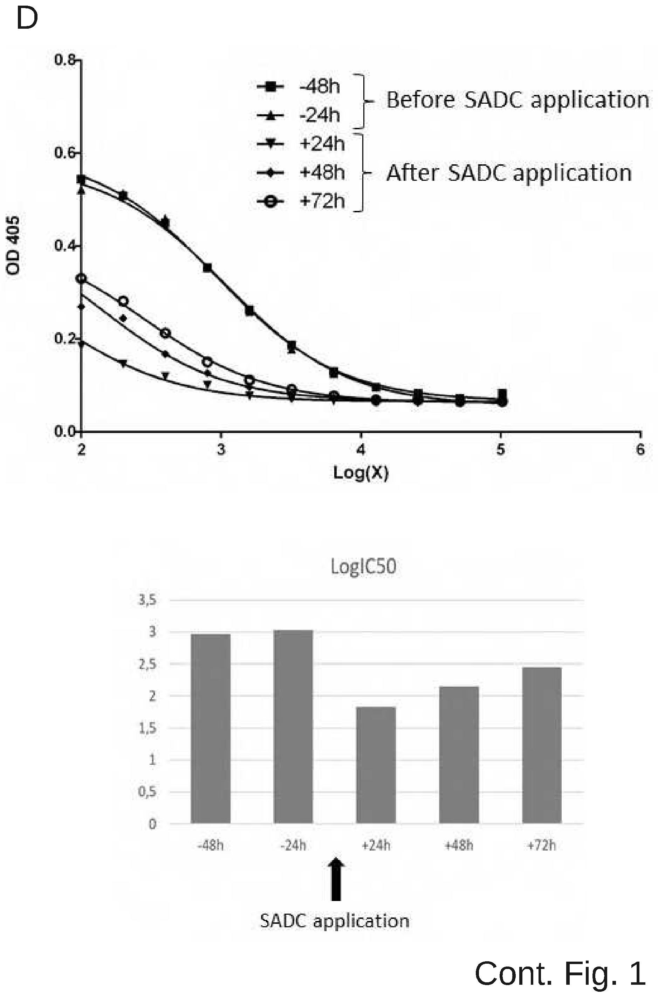

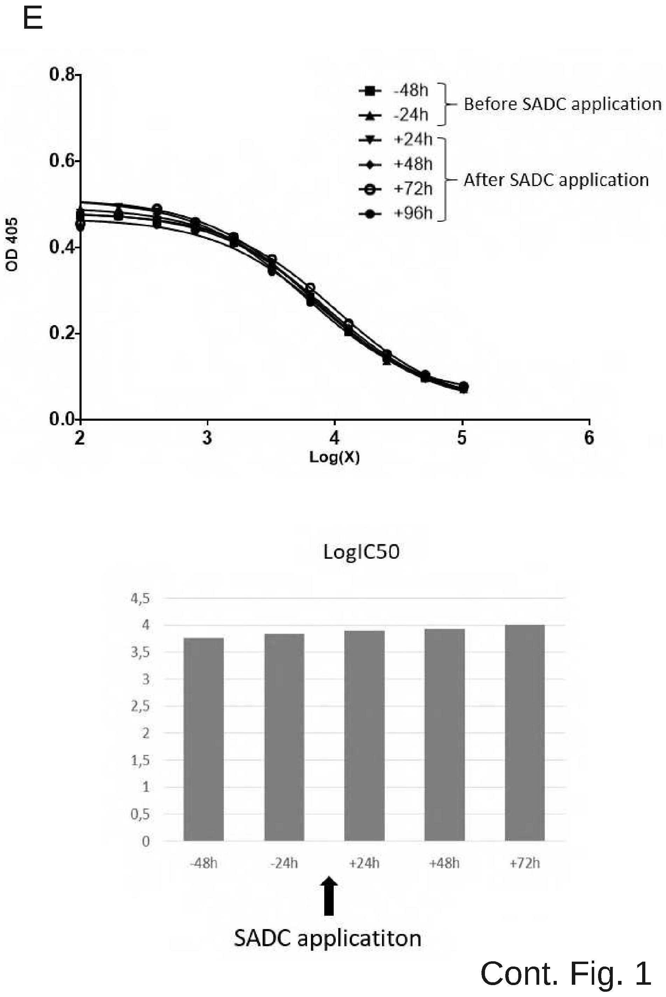

**Fig. 1****: SADCs successfully reduce the titre of undesired antibodies.** Each SADC was applied at time point 0 by i.p. injection into Balb/c mice pre-immunized by peptide immunization against a defined antigen. Each top panel shows anti-peptide titers (0.5x dilution steps; X-axis shows log(X) dilutions) against OD values (y-axis) according to a standard ELISA detecting the corresponding antibody. Each bottom panel shows titers LogIC50 (y-axis) before injection of each SADC (i.e. titers at -48h and -24h) and after application of each SADC (i.e. titers +24h, +48h and +72h after injection; indicated on the x-axis). **(A)** Compound with albumin as the biopolymer scaffold that binds to antibodies directed against EBNA1 (associated with pre-eclampsia). The mice were pre-immunized with a peptide vaccine carrying the EBNA-1 model epitope. **(B)** Compound with albumin as the biopolymer scaffold that binds to antibodies directed against a peptide derived from the human AChR protein MIR (associated with myasthenia gravis). The mice were pre-immunized with a peptide vaccine carrying the AChR MIR model epitope. **(C)** Compound with immunoglobulin as the biopolymer scaffold that binds to antibodies directed against EBNA1 (associated with pre-eclampsia). The mice were pre-immunized with a peptide vaccine carrying the EBNA-1 model epitope. **(D)** Compound with haptoglobin as the biopolymer scaffold that binds to antibodies directed against EBNA1 (associated with pre-eclampsia). The mice were pre-immunized with a peptide vaccine carrying the EBNA-1 model epitope. **(E)** Demonstration of selectivity using the same immunoglobulin-based SADC binding to antibodies directed against EBNA1 that was used in the experiment shown in panel C. The mice were pre-immunized with an unrelated amino acid sequence. No titre reduction occurred, demonstrating selectivity of the compound.

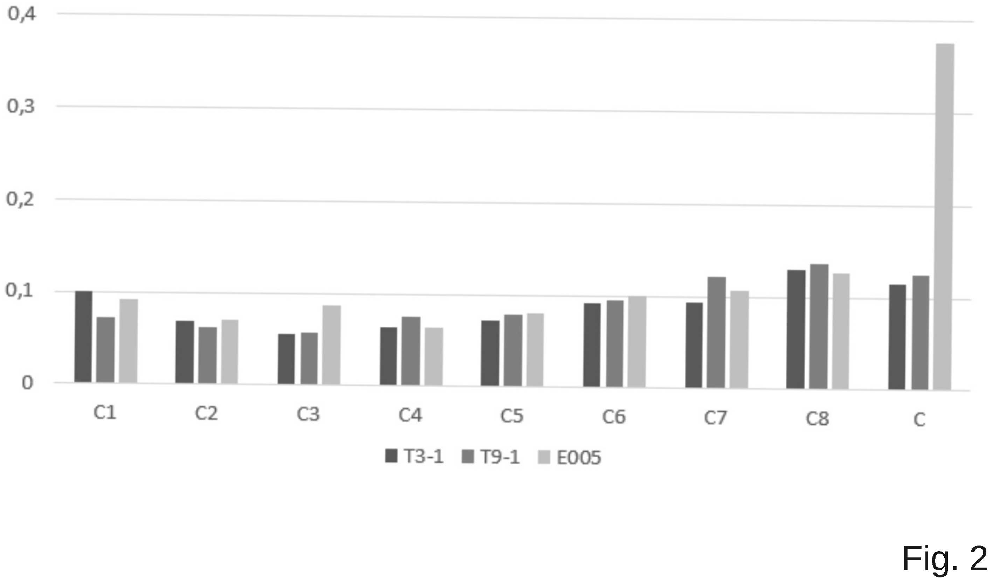

**Fig. 2****: SADCs are non-immunogenic and do not induce antibody formation after repeated injection into mice.** Animals C1-C4 as well as animals C5-C8 were treated i.p. with two different SADCs. Control animal C was vaccinated with a KLH-peptide derived from the human AChR protein MIR. Using BSA-conjugated peptide probes T3-1, T9-1 and E005 (grey bars, as indicated in the graph), respectively, for antibody titer detection by standard ELISA at a dilution of 1:100, it could be demonstrated that antibody induction was absent in animals treated with an SADC, when compared to the vaccine-treated control animal C (y-axis, OD450 nm).

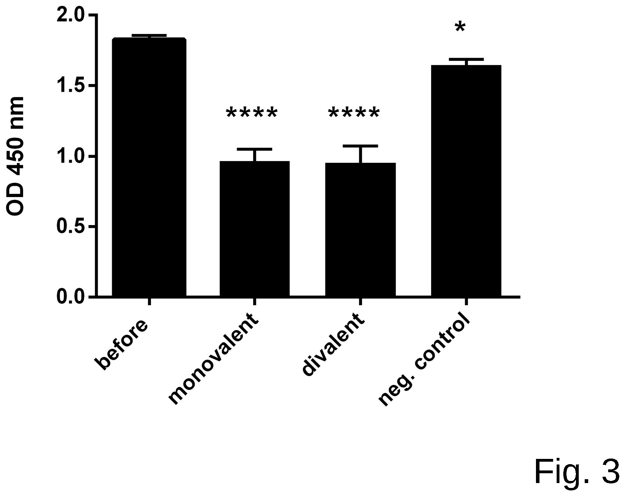

**Fig. 3****: Successful in vitro depletion of antibodies using SADCs carrying multiple copies of monovalent or divalent peptides.** SADCs with mono- or divalent peptides were very suitable to adsorb antibodies and thereby deplete them. "Monovalent" means that peptide monomers are bound to the biopolymer scaffold (i.e. n=1) whereas "divalent" means that peptide dimers are bound to the biopolymer scaffold (i.e. n=2). In the present case, the divalent peptides were "homodivalent", i.e. the peptide n-mer of the SADC is E006 - spacer - E006).

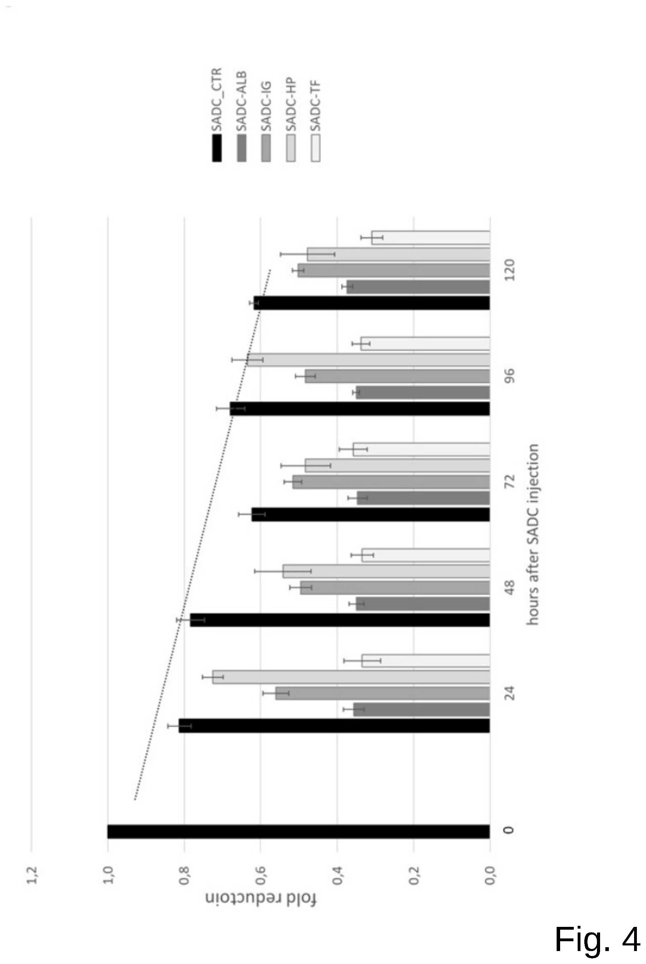

**Fig. 4****: Rapid, selective antibody depletion in mice using various SADC biopolymer scaffolds.** Treated groups exhibited rapid and pronounced antibody reduction already at 24hrs (in particular SADC-TF) when compared to the mock treated control group SADC-CTL (containing an unrelated peptide). SADC with albumin scaffold - SADC-ALB, SADC with immunoglobulin scaffold - SADC-IG, SADC with haptoglobin scaffold - SADC-HP, and SADC with transferrin scaffold - SADC-TF.

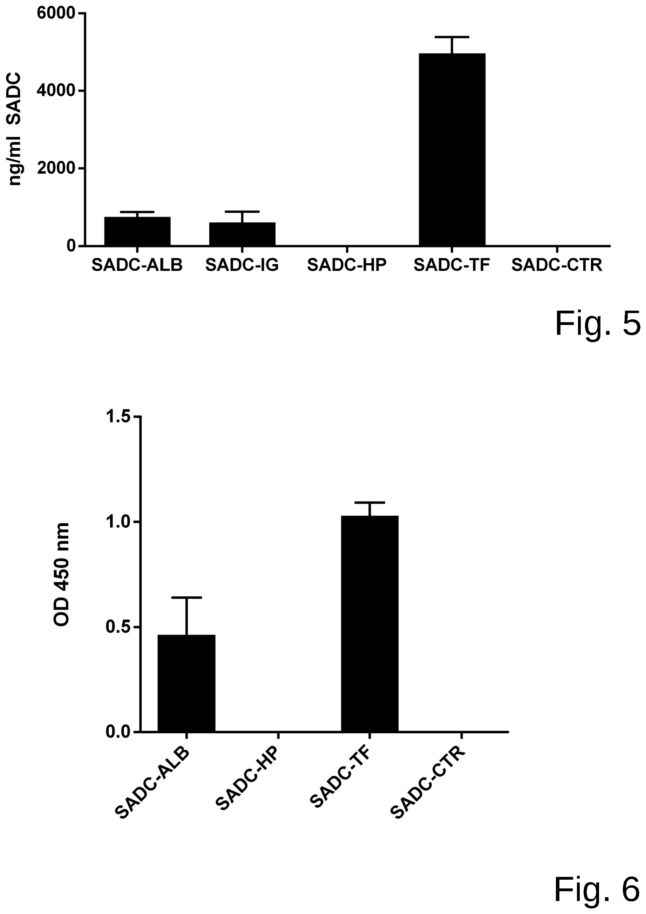

**Fig. 5****: Detection of SADCs in plasma via their peptide moieties 24hrs after SADC injection.** Both haptoglobin-scaffold-based SADCs (SADC-HP and SADC-CTL) exhibited a relatively shorter plasma half life which represents an advantage over SADCs with other biopolymer scaffolds such as SADC-ALB, SADC-IG oder SADC-TF. SADC with albumin scaffold - SADC-ALB, SADC with immunoglobulin scaffold - SADC-IG, SADC with haptoglobin scaffold - SADC-HP, and SADC with transferrin scaffold - SADC-TF.
**Fig. 6****: Detection of SADC-IgG complexes in plasma 24hrs after SADC injection.** Haptoglobin based SADCs were subject to accelerated clearance when compared to SADCs with other biopolymer scaffolds. SADC with albumin scaffold - SADC-ALB, SADC with immunoglobulin scaffold - SADC-IG, SADC with haptoglobin scaffold - SADC-HP, and SADC with transferrin scaffold - SADC-TF.
**Fig. 7****: In vitro analysis of SADC-IgG complex formation.** Animals SADC-TF and -ALB showed pronounced immunocomplex formation and binding to C1q as reflected by the strong signals and by sharp signal lowering in case 1000ng/ml SADC-TF due to the transition from antigen-antibody equilibrium to antigen excess. In contrast, in vitro immunocomplex formation with SADC-HP or SADC-IG were much less efficient when measured in the present assay. These findings corroborate the finding that haptoglobin scaffolds are advantageous over other SADC biopolymer scaffolds because of the reduced propensity to activate the complement system. SADC with albumin scaffold - SADC-ALB, SADC with immunoglobulin scaffold - SADC-IG, SADC with haptoglobin scaffold - SADC-HP, and SADC with transferrin scaffold - SADC-TF.

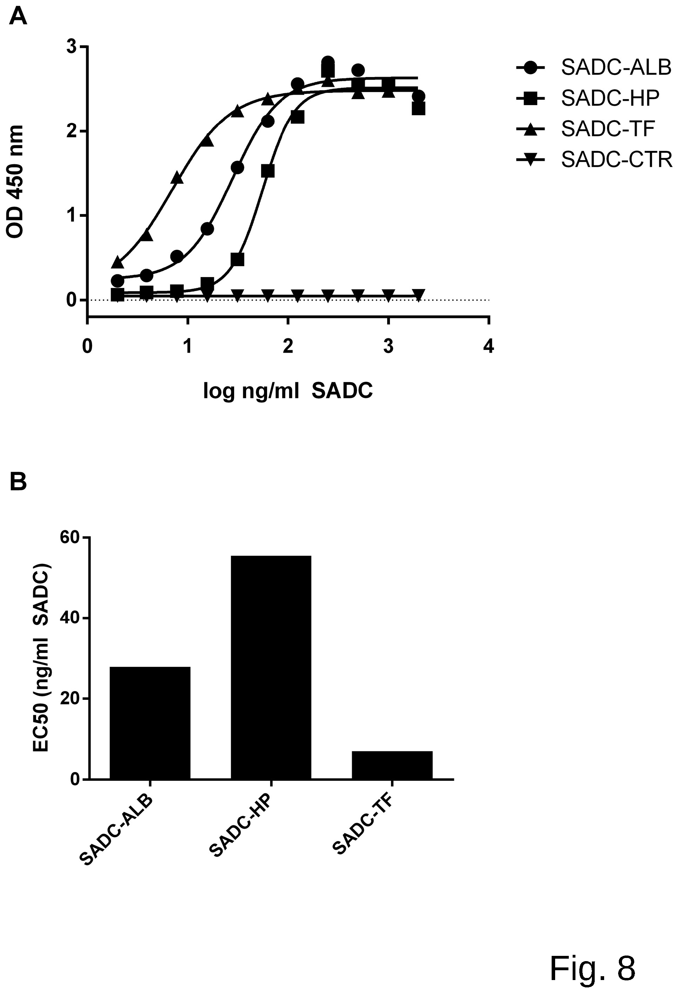

**Fig. 8****: Determination of IgG capturing by SADCs in vitro.** SADC-HP showed markedly less antibody binding capacity in vitro when compared to SADC-TF or SADC-ALB. SADC with albumin scaffold - SADC-ALB, SADC with immunoglobulin scaffold - SADC-IG, SADC with haptoglobin scaffold - SADC-HP, and SADC with transferrin scaffold - SADC-TF.

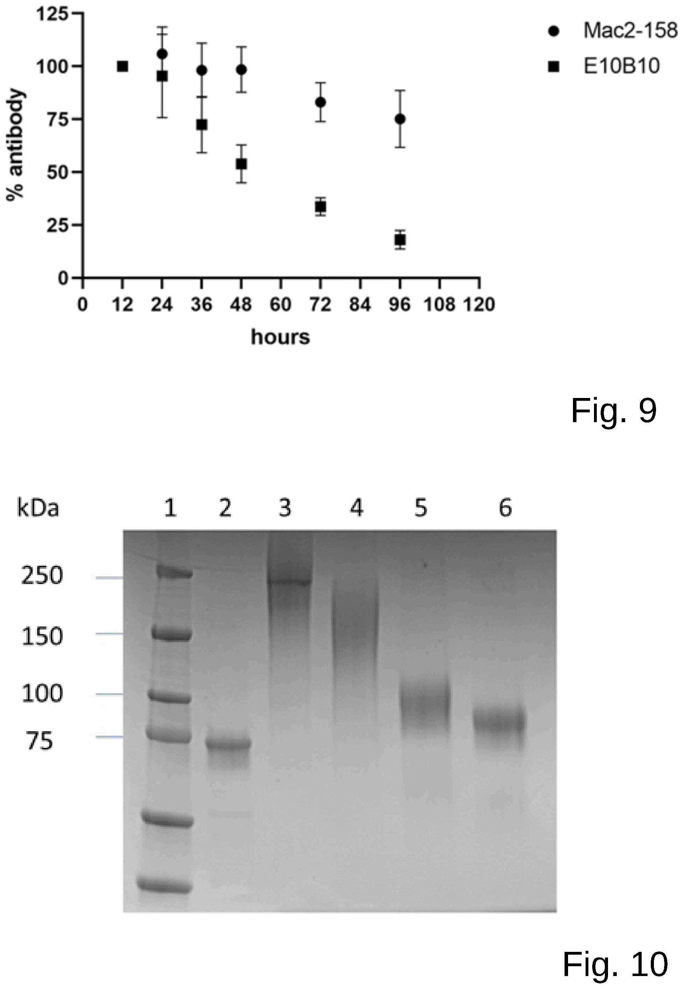

**Fig. 9****: Blood clearance of an anti-CD163-antibody-based biopolymer scaffold.** In a mouse model, mAb E10B10 (specific for murine CD163) is much more rapidly cleared from circulation than mAb Mac2-158 (specific for human CD163 but not for murine CD163, thus serving as negative control in this experiment).
**Fig. 10****: PEG was efficiently coupled to a transferrin biopolymer scaffold to yield a compound of the present invention.** 2.5 µg human apo-transferrin coupled to PEG (10 kDa, 5 kDa, 2 kDa, 750 Da) as analysed by SDS page. All conjugates show a complete shift according to the size of their conjugated PEG molecule (lanes 3-6, PEG size 10 kDa, 5 kDa, 2 kDa, 750 Da, respectively), when compared to the unconjugated apo-transferrin (lane 2) which delineates the migration of the unconjugated scaffold protein. The original human apo-transferrin band (lane 2) completely disappears upon conjugation of PEG (lanes 3-6) thereby providing a quality proof and consistency check for functional animal experiments with PEG-SADCs. Lane 1 contains a protein ladder.

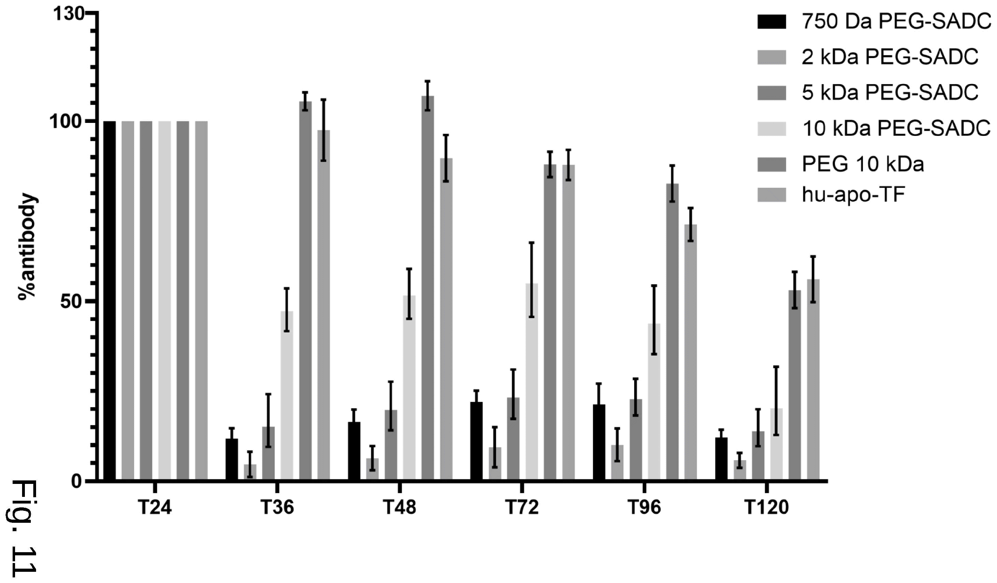

**Fig. 11****: Selective anti-PEG antibody depletion by a compound of the present invention in vivo.** Unexpectedly, 2 kDa, 5 kDa and 750 Da PEG-SADC provide effective and rapid anti-PEG antibody signal lowering. The 2 kDa PEG-SADC formulation is most effective and causes long lasting anti-PEG antibody lowering until the end of the experiment at timepoint (T) 120 hours (T120). In contrast, the 10kDa PEG-SADC formulation is less effective. Importantly, the previously suggested free PEG 10kDa formulation ("PEG 10kDa", no biopolymer scaffold) does not show any lowering of the antibody signal at the comparable dose. Neither does the biopolymer scaffold ("hu-apo-TF") alone.

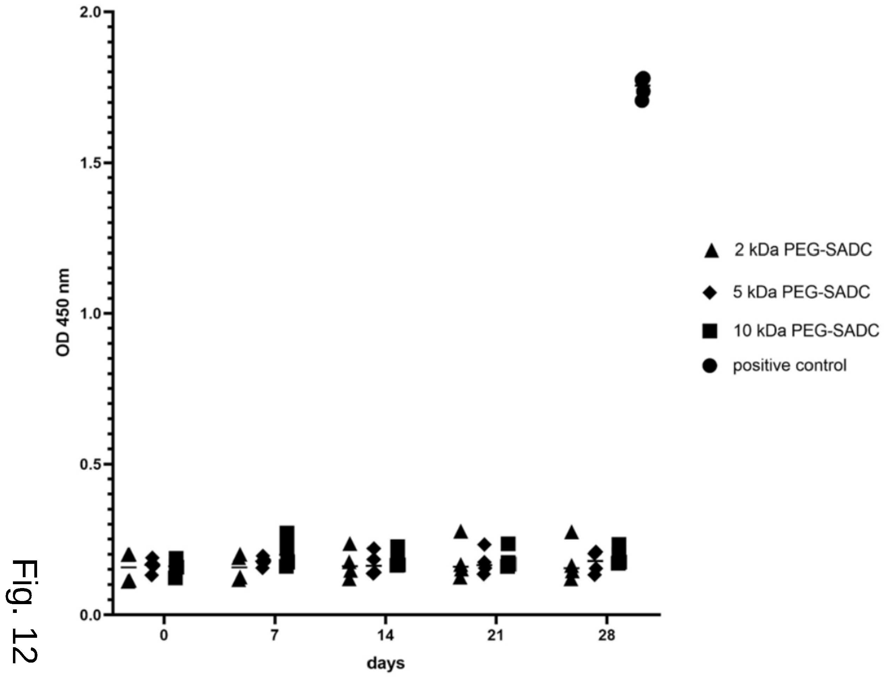

**Fig. 12****: Compounds of the present invention are non-immunogenic.** PEG-SADCs do not induce any detectable anti-PEG antibody increase after repeated injections.

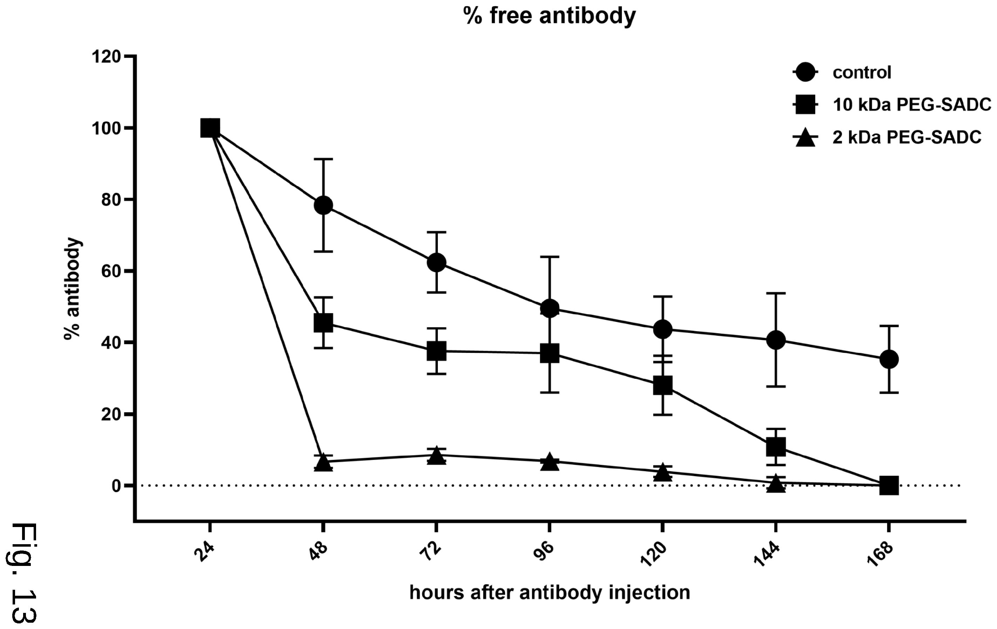

**Fig. 13****: Comparison between 2 kDa PEG-SADC and 10kDa PEG-SADC.** An independent experiment corroborates the surprising results shown in Fig. 11, over a longer observation period. The 2 kDa PEG-SADC formulation is most effective and causes long lasting anti-PEG antibody lowering. In contrast, the 10kDa PEG-SADC formulation still achieves anti-PEG antibody lowering but is less effective.

### EXAMPLES

Examples 1-8 demonstrate that SADCs are very well suited for selective removal of undesirable antibodies (as shown with antibodies binding to peptide epitopes, i.e. on the basis of peptide-based SADCs). Example 9-11 contain more details on anti-CD163 antibody scaffolds. Finally, the SADC concept was successfully extended to undesirable anti-PEG antibodies, as shown in Examples 12-16.

### Example 1: SADCs effectively reduce the titre of undesired antibodies.

*Animal models:* In order to provide *in vivo* models with measurable titers of prototypic undesired antibodies in human indications, BALB/c mice were immunized using standard experimental vaccination with KLH-conjugated peptide vaccines derived from established human autoantigens or anti-drug antibodies. After titer evaluation by standard peptide ELISA, immunized animals were treated with the corresponding test SADCs to demonstrate selective antibody lowering by SADC treatment. All experiments were performed in compliance with the guidelines by the corresponding animal ethics authorities.

*Immunization of mice with model antigens:* Female BALB/c mice (aged 8-10 weeks) were supplied by Janvier (France), maintained under a 12h light/12h dark cycle and given free access to food and water. Immunizations were performed by s.c. application of KLH carrier-conjugated peptide vaccines injected 3 times in biweekly intervals. KLH conjugates were generated with peptide T3-2 (SEQ ID NO. 33: CGRPQKRPSCIGCKG), which represents an example for molecular mimicry between a viral antigen (EBNA-1) and an endogenous human receptor antigen, namely the placental GPR50 protein, that was shown to be relevant to preeclampsia (Elliott et al.). In order to confirm the generality of this approach, a larger antigenic peptide derived from the autoimmune condition myasthenia gravis was used for immunization of mice with a human autoepitope. In analogy to peptide T3-2, animals were immunized with peptide T1-1 (SEQ ID NO. 34: LKWNPDDYGGVKKIHIPSEKGC), derived from the MIR (main immunogenic region) of the human AChR protein which plays a fundamental role in pathogenesis of the disease (Luo et al.). The T1-1 peptide was used for immunizing mice with a surrogate partial model epitope of the human AChR autoantigen. The peptide T8-1 (SEQ ID NO. 35: DHTLYTPYHTHPG) was used to immunize control mice to provide a control titer for proof of selectivity of the system. For vaccine conjugate preparation, KLH carrier (Sigma) was activated with sulfo-GMBS (Cat. Nr. 22324 Thermo), according to the manufacturer's instructions, followed by addition of either N- or C-terminally cysteinylated peptides T3-2 and T1-1 and final addition of Alhydrogel^{®} before injection into the flank of the animals. The doses for vaccines T3-2 and T1-1 were 15µg of conjugate in a volume of 100ul per injection containing Alhydrogel^{®} (InvivoGen VAC-Alu-250) at a final concentration of 1% per dose.

*Generation of prototypic SADCs:* For testing selective antibody lowering activity by SADCs of T3-2 and T1-1 immunized mice, SADCs were prepared with mouse serum albumin (MSA) or mouse immunoglobulin (mouse-Ig) as biopolymer scaffold in order to provide an autologous biopolymer scaffold, that will not induce any immune reaction in mice, or non-autologuous human haptoglobin as biopolymer scaffold (that did not induce an allogenic reaction after one-time injection within 72 hours). N-terminally cysteinylated SADC peptide E049 (SEQ ID NO. 13: GRPQKRPSCIG) and/or C-terminally cysteinylated SADC peptide E006 (SEQ ID NO. 36: VKKIHIPSEKG) were linked to the scaffold using sulfo-GMBS (Cat. Nr. 22324 Thermo)-activated MSA (Sigma; Cat. Nr. A3559) or -mouse-Ig (Sigma, I5381) or -human haptoglobin (Sigma H0138) according to the instructions of the manufacturer, thereby providing MSA-, Ig- and haptoglobin-based SADCs with the corresponding cysteinylated peptides, that were covalently attached to the lysines of the corresponding biopolymer scaffold. Beside conjugation of the cysteinylated peptides to the lysines via a bifunctional amine-to-sulfhydryl crosslinker, a portion of the added cysteinylated SADC peptides directly reacted with sulfhydryl groups of cysteins of the albumin scaffold protein, which can be detected by treating the conjugates with DTT followed by subsequent detection of free peptides using mass spectrometry or any other analytical method that detects free peptide. Finally, these SADC conjugates were dialysed against water using Pur-A-Lyzer^{™} (Sigma) and subsequently lyophilized. The lyophilized material was resuspended in PBS before injection into animals.

*In vivo functional testing of* SADCs: Prototypic SADCs, SADC-E049 and SADC-E006 were injected intraperitoneally (i.p.; as a surrogate for an intended intravenous application in humans and larger animals) into the mice that had previously been immunized with peptide vaccine T3-2 (carrying the EBNA-1 model epitope) and peptide vaccine T1-1 (carrying the AChR MIR model epitope). The applied dose was 30µg SADC conjugate in a volume of 50µl PBS. Blood takes were performed by submandibular vein puncture, before (-48h, -24h) and after (+24h,+48h,+72h, etc.) i.p. SADC injections, respectively, using capillary micro-hematocrit tubes. Using ELISA analysis (see below), it was found that both prototypic SADCs were able to clearly reduce the titers over a period of at least 72 hrs in the present animal model. It could therefore be concluded that SADCs can be used to effectively reduce titers *in vivo.*

*Titer analysis:* Peptide ELISAs were performed according to standard procedures using 96-well plates (Nunc Medisorp plates; Thermofisher, Cat Nr 467320) coated for 1h at RT with BSA-coupled peptides (30nM, dissolved in PBS) and incubated with the appropriate buffers while shaking (blocking buffer, 1% BSA, 1x PBS; washing buffer, 1xPBS / 0,1% Tween; dilution buffer, 1xPBS / 0.1% BSA /0,1% Tween). After serum incubation (dilutions starting at 1:50 in PBS; typically in 1:3 or 1:2 titration steps), bound antibodies were detected using Horseradish Peroxidase-conjugated goat anti-mouse IgG (Fc) from Jackson immunoresearch (115-035-008). After stopping the reaction, plates were measured at 450nm for 20min using TMB. EC50 were calculated from readout values using curve fitting with a 4-parameter logistic regression model (GraphPad Prism) according to the procedures recommended by the manufacturer. Constraining parameters for ceiling and floor values were set accordingly, providing curve fitting quality levels of R² >0.98.

Figure 1A shows an in vivo proof of concept in a mouse model for in vivo selective plasma-lowering activity of a prototypic albumin-based SADC candidate that binds to antibodies directed against EBNA1, as a model for autoantibodies and mimicry in preeclampsia (Elliott et al.). For these mouse experiments, mouse albumin was used, in order to avoid any reactivity against a protein from a foreign species. Antibody titers were induced in 6 months old Balb/c mice by standard peptide vaccination. The bottom panel demonstrates that titers LogIC50 (y-axis) before SADC injection (i.e. titers at -48h and -24h) were higher than titers LogIC50 after SADC application (i.e. titers +24h, +48h and +72h after injection; indicated on the x-axis).

A similar example is shown in Figure 1B, using an alternative example of a peptidic antibody binding moiety for a different disease indication. Antibody lowering activity of an albumin-based SADC in a mouse model that was pre-immunized with a different peptide derived from the human AChR protein MIR region (Luo et al.) in order to mimic the situation in myasthenia gravis. The induced antibody titers against the AChR-MIR region were used as surrogate for anti-AChR-MIR autoantibodies known to play a causative role in myasthenia gravis (reviewed by Vincent et al.). A clear titer reduction was seen after SADC application.

Figures 1C and 1D demonstrate the functionality of SADC variants comprising alternative biopolymer scaffolds. Specifically, Figure 1C shows that an immunoglobulin scaffold can be successfully used whereas Figure 1D demonstrates the use of a haptoglobin-scaffold for constructing an SADC. Both examples show an *in vivo* proof of concept for selective antibody lowering by an SADC, carrying covalently bound example peptide E049.

The haptoglobin-based SADC was generated using human Haptoglobin as a surrogate although the autologuous scaffold protein would be preferred. In order to avoid formation of antihuman-haptoglobin antibodies, only one single SADC injection per mouse of the non-autologuous scaffold haptoglobin was used for the present experimental conditions. As expected, under the present experimental conditions (i.e. one-time application), no antibody reactivity was observed against the present surrogate haptoglobin homologue.

Figure 1E demonstrates the selectivity of the SADC system. The immunoglobulin-based SADC carrying the peptide E049 (i.e. the same as in Figure 1C) cannot reduce the Ig-titer that was induced by a peptide vaccine with an unrelated, irrelevant aminoacid sequence, designated peptide T8-1 (SEQ ID NO. 35: DHTLYTPYHTHPG). The example shows an *in vivo* proof of concept for the selectivity of the system. The top panel shows anti-peptide T8-1 titers (0,5x dilution steps starting from 1:50 to 1:102400; X-axis shows log(X) dilutions) against OD values (y-axis) according to a standard ELISA. T8-1-titers are unaffected by administration of SADC-Ig-E049 after application. The bottom panel demonstrates that the initial titers LogIC50 (y-axis) *before* SADC injection (i.e. titers at -48h and -24h) are unaffected by administration of SADC-Ig-E049 (arrow) when compared to the titers LogIC50 *after* SADC application (i.e. titers +24h, +48h and +72h; as indicated on the x-axis), thereby demonstrating the selectivity of the system.

### Example 2: Immunogenicity of SADCs.

In order to exclude immunogenicity of SADCs, prototypic candidate SADCs were tested for their propensity to induce antibodies upon repeated injection. Peptides T3-1 and T9-1 were used for this test. T3-1 is a 10-amino acid peptide derived from a reference epitope of the Angiotensin receptor, against which agonistic autoantibodies are formed in a pre-eclampsia animal model (Zhou et al.); T9-1 is a 12-amino acid peptide derived from a reference anti-drug antibody epitope of human IFN gamma (Lin et al.). These control SADC conjugates were injected 8 x every two weeks i.p. into naïve, non-immunized female BALB/c mice starting at an age of 8-10 weeks.

Animals C1-C4 were treated i.p. (as described in example 1) with SADC T3-1. Animals C5-C8 were treated i.p. with an SADC carrying the peptide T9-1. As a reference signal for ELISA analysis, plasma from a control animal that was vaccinated 3 times with KLH-peptide T1-1 (derived from the AChR-MIR, explained in Example 1) was used. Using BSA-conjugated peptide probes T3-1, T9-1 and E005 (SEQ ID NO. 37: GGVKKIHIPSEK), respectively, for antibody titer detection by standard ELISA at a dilution of 1:100, it could be demonstrated that antibody induction was absent in SADC-treated animals, when compared to the vaccine-treated control animal C (see Figure 2). The plasmas were obtained by submandibular blood collection, 1 week after the 3rd vaccine injection (control animal C) and after the last of 8 consecutive SADC injections in 2-weeks intervals (animals C1-C8), respectively. Thus it was demonstrated that SADCs are non-immunogenic and do not induce antibody formation after repeated injection into mice.

### Example 3: Successful in vitro depletion of antibodies using SADCs carrying multiple copies of monovalent or divalent peptides.

Plasma of E006-KLH (VKKIHIPSEKG (SEQ ID NO: 36) with C-terminal cysteine, conjugated to KLH) vaccinated mice was diluted 1:3200 in dilution buffer (PBS + 0.1% w/v BSA + 0.1% Tween20) and incubated (100 µl, room temperature) sequentially (10 min/well) four times on single wells of a microtiter plate that was coated with 2.5 µg/ml (250 ng/well) of SADC or 5 µg/ml (500 ng/well) albumin as negative control.

In order to determine the amount of free, unbound antibody present before and after incubation on SADC coated wells, 50 µl of the diluted serum were taken before and after the depletion and quantified by standard ELISA using E006-BSA coated plates (10 nM peptide) and detection by goat anti mouse IgG bio (Southern Biotech, diluted 1:2000). Subsequently, the biotinylated antibody was detected with Streptavidin-HRP (Thermo Scientific, diluted 1:5000) using TMB as substrate. Development of the signal was stopped with 0.5 M sulfuric acid.

ELISA was measured at OD450nm (y-axis). As a result, the antibody was efficiently adsorbed by either coated mono- or divalent SADCs containing peptide E006 with C-terminal cysteine (sequence VKKIHIPSEKGC, SEQ ID NO: 36) (before=non-depleted starting material; mono- divalent corresponds to peptides displayed on the SADC surface; neg. control was albumin; indicated on the x-axis). See Fig. 3. ("Monovalent" means that peptide monomers are bound to the biopolymer scaffold (i.e. n=1) whereas "divalent" means that peptide dimers are bound to the biopolymer scaffold (i.e. n=2). In the present case, the divalent peptides were "homodivalent", i.e. the peptide n-mer of the SADC is E006 - S - E006.)

This demonstrates that SADCs with mono- or divalent peptides are very suitable to adsorb antibodies and thereby deplete them.

### Example 4: Rapid, selective antibody depletion in mice using various SADC biopolymer scaffolds.

10 µg of model undesired antibody mAB anti V5 (Thermo Scientific) was injected i.p. into female Balb/c mice (5 animals per treatment group; aged 9-11 weeks) followed by intravenous injection of 50 µg SADC (different biopolymer scaffolds with tagged V5 peptides bound, see below) 48hrs after the initial antibody administration. Blood was collected at 24hrs intervals from the submandibular vein. Blood samples for time point 0 hrs were taken just before SADC administration.

Blood was collected every 24 hrs until time point 120 hrs after the SADC administration (x-axis). The decay and reduction of plasma anti-V5 IgG levels after SADC administration was determined by anti V5 titer readout using standard ELISA procedures in combination with coated V5-peptide-BSA (peptide sequence IPNPLLGLDC - SEQ ID NO: 57) and detection by goat anti mouse IgG bio (Southern Biotech, diluted 1:2000) as shown in Fig. 4. In addition, SADC levels (see Example 6) and immunocomplex formation (see Example 7) were analyzed.

EC50[OD450] values were determined using 4 parameter logistic curve fitting and relative signal decay between the initial level (set to 1 at time point 0) and the following time points (x-axis) was calculated as ratio of the EC50 values (y-axis, fold signal reduction EC50). All SADC peptides contained tags for direct detection of SADC and immunocomplexes from plasma samples; peptide sequences used for SADCs were: IPNPLLGLDGGSGDYKDDDDKGK(SEQ ID NO: 38)-(BiotinAca)GC (SADC with albumin scaffold - SADC-ALB, SADC with immunoglobulin scaffold - SADC-IG, SADC with haptoglobin scaffold - SADC-HP, and SADC with transferrin scaffold - SADC-TF) and unrelated peptide VKKIHIPSEKGGSGDYKDDDDKGK(SEQ ID NO: 39)-(BiotinAca)GC as negative control SADC (SADC-CTR).

The SADC scaffolds for the different treatment groups of 5 animals are displayed in black/grey shades (see inset of Fig. 4).

Treated groups exhibited rapid and pronounced antibody reduction already at 24hrs (in particular SADC-TF) when compared to the mock treated control group SADC-CTL. SADC-CTR was used as reference for a normal antibody decay since it has no antibody lowering activity because its peptide sequence is not recognized by the administered anti V5 antibody. The decay of SADC-CTR is thus marked with a trend line, emphasizing the antibody level differences between treated and mock treated animals.

In order to determine the effectivity of selective antibody lowering under these experimental conditions, a two-way ANOVA test was performed using a Dunnett's multiple comparison test. 48 hrs after SADC administration, the antibody EC50 was highly significantly reduced in all SADC groups (p<0.0001) compared to the SADC-CTR reference group (trend line). At 120 hrs after SADC administration, antibody decrease was highly significant in the SADC-ALB and SADC-TF groups (both p<0.0001) and significant in the SADC-HP group (p=0.0292), whereas the SADC-IG group showed a trend towards an EC50 reduction(p = 0.0722) 120 hrs after SADC administration. Of note, selective antibody reduction was highly significant (p<0.0001) in the SADC-ALB and SADC-TF groups at all tested time-points after SADC administration.

It is concluded that all SADC biopolymer scaffolds were able to selectively reduce antibody levels. Titer reduction was most pronounced with SADC-ALB and SADC-TF and no rebound or recycling of antibody levels was detected towards the last time points suggesting that undesired antibodies are degraded as intended.

### Example 5: Detection of SADCs in plasma 24hrs after SADC injection.

Plasma levels of different SADC variants at 24hrs after i.v. injection into Balb/c mice. Determination of Plasma levels (y-axis) of SADC-ALB, -IG, -HP, -TF and the negative control SADC-CTR (x-axis), were detected in the plasmas from the animals already described in example 5. Injected plasma SADC levels were detected by standard ELISA whereby SADCs were captured via their biotin moieties of their peptides in combination with streptavidin coated plates (Thermo Scientific). Captured SADCs were detected by mouse anti Flag-HRP antibody (Thermo Scientific, 1:2,000 diluted) detecting the Flag-tagged peptides (see also example 7):
Assuming a theoretical amount in the order of 25 µg/ml in blood after injecting 50 µg SADC i.v., the detectable amount of SADC ranged between 799 and 623 ng/ml for SADC-ALB or SADC-IG and up to approximately 5000 ng/ml for SADC-TF, 24 hrs after SADC injection. However surprisingly and in contrast, SADC-HP and control SADC-CTR (which is also a SADC-HP variant, however carrying the in this case unrelated negative control peptide E006, see previous examples), had completely disappeared from circulation 24hrs after injection, and were not detectable anymore. See Fig. 5.

This demonstrates that both Haptoglobin scaffold-based SADCs tested in the present example ((namely SADC-HP and SADC-CTR) exhibit a relatively shorter plasma half-life which represents an advantage over SADCs such as SADC-ALB, SADC-IG oder SADC-TF in regard of their potential role in complement-dependent vascular and renal damage due to the in vivo risk of immunocomplex formation. Another advantage of SADC-HP is the accelerated clearance rate of their unwanted target antibody from blood in cases where a rapid therapeutic effect is needed. The present results demonstrate that Haptoglobin-based SADC scaffolds (as represented by SADC-HP and SADC-CTR) are subject to rapid clearance from the blood, regardless of whether SADC-binding antibodies are present in the blood, thereby minimizing undesirable immunocomplex formation and showing rapid and efficient clearance. Haptoglobin-based SADCs such as SADC-HP in the present example thus provide a therapeutically relevant advantage over other SADC biopolymer scaffolds, such as demonstrated by SADC-TF or SADC-ALB, both of which are still detectable 24hrs after injection under the described conditions, in contrast to SADC-HP or SADC-CTR which both are completely cleared 24hrs after injection.

### Example 6: Detection of SADC-IgG complexes in plasma 24hrs after SADC injection.

In order to determine the amount IgG bound to SADCs in vivo, after i.v. injection of 10 µg anti V5 IgG (Thermo Scientific) followed by injection of SADC-ALB, -HP, -TF and -CTR (50 µg) administered i.v. 48h after antibody injection, plasma was collected from the submandibular vein, 24hrs after SADC injection, and incubated on streptavidin plates for capturing SADCs from plasma via their biotinylated SADC-V5-peptide [IPNPLLGLDGGSGDYKDDDDKGK(SEQ ID NO: 38) (BiotinAca)GC or in case of SADC-CTR the negative control peptide VKKIHIPSEKGGSGDYKDDDDKGK(SEQ ID NO: 39) (BiotinAca)GC]. IgG bound to the streptavidin-captured SADCs was detected by ELISA using a goat anti mouse IgG HRP antibody (Jackson Immuno Research, diluted 1:2,000) for detection of the SADC-antibody complexes present in plasma 24hrs after SADC injection. OD450nm values (y-axis) obtained for a negative control serum from untreated animals were subtracted from the OD450nm values of the test groups (x-axis) for background correction.

As shown in Fig. 6, pronounced anti-V5 antibody signals were seen in case of SADC-ALB and SADC-TF injected mice (black bars represent background corrected OD values at a dilution of 1:25 , mean value of 5 mice; standard deviation error bars), whereas no antibody signal could be detected in plasmas from SADC-HP or control SADC-CTR injected animals (SADC-CTR is a negative control carrying the irrelevant peptide bio-FLG-E006 [VKKIHIPSEKGGSGDYKDDDDKGK(SEQ ID NO: 39) (BiotinAca)GC] that is not recognized by any anti V5 antibody). This demonstrates the absence of detectable amounts of SADC-HP/IgG complexes in the plasma 24hrs after i.v. SADC application.

SADC-HP is therefore subject to accelerated clearance in anti V5 pre-injected mice when compared to SADC-ALB or SADC-TF.

### Example 7: In vitro analysis of SADC-immunoglobulin complex formation

SADC-antibody complex formation was analyzed by pre-incubating 1 µg/ml of human anti V5 antibody (anti V5 epitope tag [SV5-P-K], human IgG3, Absolute Antibody) with increasing concentrations of SADC-ALB, -IG, -HP, -TF and -CTR (displayed on the x-axis) in PBS +0.1% w/v BSA + 0.1% v/v Tween20 for 2 hours at room temperature in order to allow for immunocomplex formation in vitro. After complex formation, samples were incubated on ELISA plates that had previously been coated with 10 µg/ml of human C1q (CompTech) for 1 h at room temperature, in order to allow capturing of in vitro formed immunocomplexes. Complexes were subsequently detected by ELISA using anti human IgG (Fab specific)-Peroxidase (Sigma, diluted 1:1,000). Measured signals at OD450 nm (y-axis) reflect Antibody-SADC complex formation in vitro.

As shown in Fig. 7, SADC-TF and -ALB showed pronounced immunocomplex formation and binding to C1q as reflected by the strong signals and by sharp signal lowering in case 1000ng/ml SADC-TF due to the transition from antigen-antibody equilibrium to antigen excess. In contrast, in vitro immunocomplex formation with SADC-HP or SADC-IG were much less efficient when measured in the present assay.

Together with the in vivo data (previous examples), these findings corroborate the finding that haptoglobin scaffolds are advantageous over other SADC biopolymer scaffolds because of the reduced propensity to activate the complement system. In contrast, SADC-TF or SADC-ALB show higher complexation, and thereby carry a certain risk of activating the C1 complex with initiation of the classical complement pathway (a risk which may be tolerable in some settings, however).

### Example 8: Determination of IgG capturing by SADCs in vitro

Immunocomplexes were allowed to form in vitro, similar to the previous example, using 1 µg/ml mouse anti V5 antibody (Thermo Scientific) in combination with increasing amounts of SADCs (displayed on the x-axis). SADC-antibody complexes were captured on a streptavidin coated ELISA plate via the biotinylated SADC-peptides (see previous examples), followed by detection of bound anti-V5 using anti mouse IgG-HRP (Jackson Immuno Research, diluted 1:2,000).

Under these assay conditions, SADC-HP showed markedly less antibody binding capacity in vitro when compared to SADC-TF or SADC-ALB (see Fig. 8, A). The calculated EC50 values for IgG detection on SADCs were 7.0 ng/ml, 27.9 ng/ml and 55.5 ng/ml for SADC-TF, -ALB and -HP, respectively (see Fig. 8, B).

This in vitro finding is consistent with the observation (see previous examples) that SADC-HP has a lower immunocomplex formation capacity when compared to SADC-TF or SADC-ALB which is regarded as a safety advantage with respect to its therapeutic use for the depletion of unwanted antibodies.

### Example 9: In-vivo function of anti-CD163-antibody-based SADC biopolymer scaffold

Rapid *in vivo* blood clearance of anti-mouse-CD163 mAB E10B10 (as disclosed in WO 2011/039510 A2). mAB E10B10 was resynthesized with a mouse IgG2a backbone. 50 µg mAb E10B10 and Mac2-158 (human-specific anti-CD163 mAb as disclosed in WO 2011/039510 A2, used as negative control in this example since it does not bind to mouse CD163) were injected i.v. into mice and measured after 12, 24, 36, 48 , 72, 96 hours in an ELISA to determine the blood clearance.

mAb E10B10 was much more rapidly cleared from circulation than control mAb Mac2-158 was, as shown in Fig. 9, since E10B10 binds to the mouse CD163 whereas Mac2-158 is human-specific, although both were expressed as mouse IgG2a isotypes for direct comparison.

In conclusion, anti-CD163 antibodies are highly suitable as SADC scaffold because of their clearance profile. SADCs with such scaffolds will rapidly clear undesirable antibodies from circulation.

*Detailed methods:* 50 ug of biotinylated monoclonal antibodies E10B10 and biotinylated Mac2-158 were injected i.v. into mice and measured after 12, 24, 36, 48, 72, 96 hours to determine the clearance by ELISA: Streptavidin plates were incubated with plasma samples diluted in PBS + 0.1%BSA + 0.1% Tween20 for 1 h at room temperature (50 µl/well). After washing (3x with PBS + 0.1% Tween20), bound biotinylated antibodies were detected with anti-mouse IgG+IgM-HRP antibody at a 1:1000 dilution. After washing, TMB substrate was added and development of the substrate was stopped with TMB Stop Solution. The signal at OD450 nm was read. The EC50 values were calculated by nonlinear regression using 4 parametric curve fitting with constrained curves and least squares regression. EC50 values at time-point T12 (this was the first measured time-point after antibody injection) was set at 100%, all other EC50 values were compared to the levels at T12.

### Example 10: Epitope mapping of anti-CD163 mAbs

mAB E10B10 provides CD163-mediated, accelerated in vivo clearance from blood in mice (see example 11). The epitope of this antibody was fine mapped using circular peptide arrays, whereby the peptides were derived from mouse CD163. As a result, a peptide cluster that is recognized by mAB E10B10 was identified (see example 13).

The same epitope mapping procedure using circularized peptides was performed with mAB Mac2-158 (as disclosed in WO 2011/039510 A2). Epitope mapping results for mAB Mac2-158 yielded two peptide clusters (see example 13) which allowed further demarcation of CD163 epitope regions that are especially relevant to internalization of ligands and antibodies that bind to the receptor.

These newly characterized epitopes for Mac2-158 and E10B10 thus revealed three preferred binding regions for antibodies against CD163. Based on the fine epitope mapping work, linear or preferentially circular peptides are synthesized and used for the induction, production and selection of polyclonal or monoclonal antibodies or other CD163-binding SADC scaffolds that target CD163.

### Example 11: Epitope mapping of anti-CD163 mAbs

Peptides aligned to SRCR domain 1 of human CD163 were selected from the top 20 peptide hits of mAB Mac2-158 circular epitope mapping peptides and the most preferred sequences were selected from two peptide alignment clusters at the N-terminus and at the C-terminus of SRCR-1 of human CD163. As a result, the following sequences (as well as motifs derived therefrom) are highly suitable epitopes anti-CD163 antibodies and fragments thereof used as SADC biopolymer scaffold:
Peptide cluster 1:

| | | |
|---|---|---|
| 04 | ----------------EWGTVCNNGWSME------- | (SEQ ID NO: 7) |
| 07 | -----CSGRVEVKVQEEW------------------ | (SEQ ID NO: 40) |
| 09 | --------------QEEWGTVCNNGWS--------- | (SEQ ID NO: 8) |
| 12 | -----------------WGTVCNNGWSMEA------ | (SEQ ID NO: 9) |
| 14 | ---------------EEWGTVCNNGWSM-------- | (SEQ ID NO: 10) |
| 18 | -------------VQEEWGTVCNNGW---------- | (SEQ ID NO: 11) |
| 19 | ----------------EWGTVCNNGW---------- | (SEQ ID NO: 12) |
| 20 | -----------------WGTVCNNGWS--------- | (SEQ ID NO: 5) |
| huCD163-domain 1-3 | DGENKCSGRVEVKVQEEWGTVCNNGWSMEAVSVICN | (SEQ ID NO: 41) |

Peptide cluster 2:

| | | |
|---|---|---|
| 01 | ------------ESALWDC-------------- | (SEQ ID NO: 15) |
| 02 | ---------RGNESALWDC-------------- | (SEQ ID NO: 16) |
| 03 | -------SCRGNESALW---------------- | (SEQ ID NO: 17) |
| 05 | ------VSCRGNESALWDC-------------- | (SEQ ID NO: 18) |
| 06 | --------------ALWDCKHDGW--------- | (SEQ ID NO. 19) |
| 08 | ----DHVSCRGNESALW---------------- | (SEQ ID NO. 20) |
| 11 | --------CRGNESALWD--------------- | (SEQ ID NO. 21) |
| 13 | -----------NESALWDCKHDGW--------- | (SEQ ID NO. 22) |
| 17 | ------------ESALWDCKHDGWG-------- | (SEQ ID NO. 23) |
| huCD163-domain1-3 | RIWMDHVSCRGNESALWDCKHDGWGKHSNCTHQ | (SEQ ID NO: 42) |

Fine epitope mapping of mAb E10B10 was performed as for Mac2-158. 1068 circular peptides (sized 7, 10 and 13 amino acids) and derived from SRCR-1 to -3 of the mouse CD163 sequence (UniProKB Q2VLH6.2) were screened with mAB E10B10 and the following top binding peptides were obtained (ranked by relative signal strength). The human CD163 sequence was aligned to this cluster, revealing another highly suitable epitope:
Peptide cluster 3:

| | | |
|---|---|---|
| 01 | ---------------------VTNAPGEMKKELR--------- | (SEQ ID NO: 43) |
| 02 | ------------------ASAVTNAPGEMKK------------ | (SEQ ID NO: 44) |
| 03 | ---------------------VTNAPGEMKK------------ | (SEQ ID NO: 45) |
| 04 | ---------------------VTNAPGE--------------- | (SEQ ID NO: 46) |
| 05 | ----------------GSASAVTNAPGEM-------------- | (SEQ ID NO: 47) |
| 06 | --------------------AVTNAPGEMKKEL---------- | (SEQ ID NO: 48) |
| 07 | -----------------SASAVTNAPGEMK------------- | (SEQ ID NO: 49) |
| 08 | ---------------SGSASAVTNAPGE--------------- | (SEQ ID NO: 50) |
| 09 | --------------------AVTNAPGEMK------------- | (SEQ ID NO: 51) |
| 10 | -------------------SAVTNAPGEM-------------- | (SEQ ID NO: 52) |
| 11 | ------------------ASAVTNAPGE--------------- | (SEQ ID NO: 53) |
| 12 | -------------------SAVTNAPGEMKKE----------- | (SEQ ID NO: 54) |
| 13 | ----------------------TNAPGEMKKE----------- | (SEQ ID NO: 55) |
| mCD163 (SRCR-1, N-terminus) | VTNAPGEMKKELRLAGGENNCS | (SEQ ID NO: 56) |
| hCD163 (SRCR-1, N-terminus) | SSLGGTDKELRLVDGENKCS | (SEQ ID NO: 24) |

The human homologues of mouse peptides 01 - 13 from cluster 3 have the following sequences of the N-terminal portion of the mature human CD163 protein (UniProtKB: Q86VB7):

| Cluster 3 peptides (mouse): | human homologues: | |
|---|---|---|
| 01 | SSLGGTDKELR | (SEQ ID NO: 25) |
| 06 | SSLGGTDKEL | (SEQ ID NO: 28) |
| 12,13 | SSLGGTDKE | (SEQ ID NO: 29) |
| 02,03 | SSLGGTDK | (SEQ ID NO: 30) |
| 07,09 | SSLGGTD | (SEQ ID NO: 31) |
| 05,10 | SSLGGT | (SEQ ID NO: 32) |
| 04,08,11 | SSLGG | (SEQ ID NO: 26) |
| hCD163 (SRCR-1) | SSLGGTDKELRLVDGENKCS | (SEQ ID NO: 24) |

These homologue peptides represent further highly suitable epitopes for the anti-CD163 antibody scaffold.

### Example 12: Production and quality analysis of a compound of the present invention

Conjugation of human apo-transferrin (i.e. the biopolymer scaffold) to PEG: 9 nmol human apo-transferrin (Sigma Aldrich) were incubated with 567 nmol PEG in 50 mM HEPES buffer pH 8 for 4 hours at room temperature while shaking. 4 PEG sizes were used for the conjugation: Methoxy-PEG-NHS 10 kDa (RAPP Polymere), Methoxy-PEG-NHS 5 kDa (RAPP Polymere), Methoxy-PEG-NHS 2 kDa (RAPP Polymere) and Methoxy-PEG-NHS 750 Da (RAPP Polymere). After incubation, buffer was exchanged to 1x PBS using Amicon^{®} Ultra-4 Centrifugal Filter Units - 50 kDa cut off (Sigma Aldrich) and the protein concentration was measured using a Qubit 4 Fluorometer (Thermo Scientific).

Quality control of coupled human apo-transferrin: The coupled protein conjugates were quality checked by SDS page analysis. 2.5 µg protein was loaded onto a 4-15% gradient TGX gel under reducing conditions following SDS page analysis (250 V, approximately 20 min). Proteins were visualized with PageBlue^{™} Protein Staining Solution (Thermo Scientific).

Figure 10 shows 2.5 µg protein coupled to PEG (10 kDa, 5 kDa, 2 kDa, 750 Da) analysed by SDS page. All conjugates showed a complete shift according to the size of their conjugated PEG molecule (lanes 3-6), when compared to the unconjugated Apo-transferrin (lane 2) which delineates the migration of the unconjugated scaffold protein. The original human Apo-transferrin band (lane 2) completely disappeared upon conjugation of PEG (different sizes indicated in lanes 3-6) thereby providing a quality proof and consistency check for functional animal experiments with PEG-SADCs.

In conclusion, PEG was efficiently coupled to human apo-transferrin to produce a compound of the present invention.

### Example 13: Selective anti-PEG antibody depletion by a compound of the present invention in vivo

4 female Balb/c mice per group, typically aged 9-11 weeks, were injected with 10 µg rabbit anti-PEG antibody (abcam, ab190652) followed by injection of 50µg PEG-SADC (see example 12 for production method) in a volume of 100 µl at timepoint (T) 24 hrs. It was previously determined for this experiment that the half-life of rabbit IgG was in the range of 5 days. Blood was taken at different time points and anti-PEG antibody detected by ELISA.

Unexpectedly, it was found that 2 kDa, 5 kDa and 750 Da PEG-SADC provided effective and rapid anti-PEG antibody signal lowering. The 2 kDa PEG-SADC formulation was most effective and long lasting anti PEG antibody lowering until the end of the experiment at timepoint (T) 120 hours (T120). In contrast, the 10kDa PEG-SADC formulation was less effective. Importantly, the previously suggested free PEG 10kDa formulation (cf. WO 2019/046185 A1 and McSweeney et al.) did not show any lowering of the antibody signal at the comparable dose. As a reference, relative anti PEG antibody levels were set to 100% just before SADC application and deduced from a reference antibody concentration (2-fold titration of antibody starting at 200 ng/ml) on each plate of the ELISA. Bars indicate the geometric mean, and error bars correspond to geometric standard deviation. Figure 11 shows efficient antibody lowering with 750 Da PEG-SADC, 2 kDa PEG-SADC and 5 kDa PEG-SADCs, while the 10kDa PEG-SADC formulation was less efficient. The PEG-only formulation (PEG 10kDa) did not show any lowering of the antibody at the comparable dose used (human Apo-transferrin was used as negative control and demarcate the natural decay of rabbit IgG in mice at approximately 5 days).

*Detailed methods:* PEG-SADC preparations were resuspended in PBS and injected intravenously, typically 24 hours after initial application of anti-PEG antibody (abcam, ab190652). The applied antibody dose was 10ug in 100 ul PBS (unless otherwise indicated). Blood takes were performed by submandibular vein puncture, before (indicated by negative hr numbers) or after (indicated by positive hr numbers) SADC injections. The effectivity of selective antibody lowering was measured by standard ELISA using biotinylated PEG (20kDa PEG-Biotin, Creative PEGWorks) on streptavidin plates (Pierce^{™} Streptavidin Coated High Capacity Plates, Clear, 96-Well, Thermo Scientific). Upon incubation of the serum on the plates (diluted in PBS + 0.1% BSA + 0.01% Tween20), plates were washed with PBS + 0.01% Tween 20. Bound antibodies were detected with anti-rabbit IgG-HRP (Peroxidase AffiniPure Goat Anti-Rabbit IgG, Fc fragment specific, Jackson Immuno Research). After washing, TMB was used as a substrate and the development reaction was stopped with TMB Stop solution. Signals were read at 450 nm.

### Example 14: Compounds of the present invention are non-immunogenic

The aim was to determine whether the intravenous injection of PEG-SADCs would lead to an increase of anti-PEG antibodies in the body. Three female Balb/c mice (aged 9-11 weeks) were injected with PEG-SADCs with different PEG chain lengths. The immunization protocol for PEG-SADCs was a weekly i.v. injection of 50µg PEG-SADC. In total, 4 injections were performed for each PEG-SADC. Blood was collected before the first injection, as well as one week after each injection.

The analysis of anti-PEG IgG and IgM was performed by ELISA against 20kDa PEG-Biotin (Creative PEGWorks). 2 µg/ml 20kDa PEG-Biotin were bound to streptavidin-plates (Pierce^{™} Streptavidin Coated High Capacity Plates, Clear, 96-Well, Thermo Scientific) for 1 h. As a positive control, 2 µg/ml biotinylated mouse IgM was immobilized on the Streptavidin plate (Sigma). Biotinylation of the mouse IgM antibody was performed by using the EZ-Link^{™} NHS-PEG4 Biotinylation Kit (Thermo Scientific). After washing the plates 3 times with PBS + 0.01% Tween20, mouse sera were incubated on the plate at a dilution of 1:100 (in PBS + 0.1% BSA + 0.01% Tween20) in triplicates. Bound mouse antibodies were detected with goat anti mouse IgG+IgM-HRP antibody (Jackson Immuno Research) at a dilution of 1:1000. After washing, TMB was used as a substrate and the development reaction was stopped with TMB Stop solution. Signals were read at 450 nm.

In Fig. 12, mean values (OD 450 nm values) for individual mice are shown as a scatter dot blot, including 4 signals of a positive control in order to highlight the saturation range of this ELISA as a technical control.

PEG-SADCs did not induce any detectable anti-PEG antibody increase after repeated injections of PEG-SADCs (cf. Fig. 12). This underscores the suitability of the inventive PEG-SADCs to reduce undesirable anti-PEG antibodies in vivo, even when administrated repeatedly.

### Example 15: Increased PEG-coupling density by using a peptide-based linker

It was tested whether a peptide-based linker might provide improve PEG conjugation to the biopolymer scaffold.

A biopolymer scaffold (human apo-transferrin) was incubated with a 75-molar excess of Sulfo-GMBS (N-γ-maleimidobutyryl-oxysulfosuccinimide ester) for 1 hour at room temperature, excess Sulfo-GMBS was removed by gel filtration. Subsequently, a 40-molar excess of peptide CGK(Biotin-Aca)GGGGNPGY-NH2 (SEQ ID NO: 58) was added and the mixture incubated for 1 hour at room temperature (Aca is epsilon-aminocaproic acid). Excess spacer peptide was removed by ultrafiltration using a 50-kDa-cut-off membrane. Subsequently, an 80-molar excess of 2 kDa NHS-PEG was added and the mixture incubated for 1 hour at room temperature to yield the final compound S3, analyzed in duplicate (sample 3, 4; see table 3).

For comparison, the biopolymer scaffold (human apo-transferrin) was incubated with an 80-molar excess of 2 kDa NHS-PEG (1 hour, room temperature) to yield compound S5 (analyzed in duplicate).

Further comparison constructs S1, S2 and S4 were created (see Table 3 below).

To determine the amount of covalently bound PEG molecules, size exclusion chromatography with inline multi-angle light scattering (SEC-MALS) was performed as follows: Prior to injection, samples were spun at 10,000 g for 10 min. Using a Malvern Panalytical OMNISEC system, samples were injected onto a Superdex 200 increase 10/300 GL size exclusion column at 0.5 ml/min. PBS was used as a running buffer. BSA was used to calibrate and confirm the detection system which consisted of refractive index, UV/Vis and light scattering detectors. Data were analyzed with OMNISEC version 5.12 software using the protein conjugate analysis method.

Surprisingly, it turned out that a peptide-based linker was suitable to increase PEG-coupling density while maintaining the antibody-depleting functionality of the compound. Table 3 (see below) shows that by using such a linker based on a short peptide, the amount of PEG conjugated to the biopolymer scaffold was significantly increased, by approx. 68% (see comparison between compounds S3 and S5).

**Table 3**

| construct | sample | peptide | PEG | GMBS | total mass [Da] | protein mass [Da] | peptide mass [Da] | #peptides | PEG mass [Da] | #PEG | average #PEGs |
|---|---|---|---|---|---|---|---|---|---|---|---|
| S1 | 1 | no peptide | - | + | 83590 | | | | | | |
| S2 | 2 | peptide | - | + | 108300 | 108300 | 24710 | 18,18 | | | |
| S3 | 3 | peptide | + | + | 131675 | 107841,83 | | | 23833,18 | 11,92 | **12,27** |
| S3 | 4 | peptide | + | + | 129449 | 104206,45 | | | 25242,56 | 12,62 | |
| S4 | 5 | no peptide | | - | 82770 | | | | | | |
| S5 | 6 | no peptide | + | - | 96643 | 81276,76 | | | 15366,24 | 7,68 | 7,33 |
| S5 | 7 | no peptide | + | - | 96269 | 82310 | | | 13959,01 | 6,98 | |

### Example 16: Comparison between 2 kDa PEG-SADC and 10kDa PEG-SADC

50 µg 2 kDa- and 10 kDa-methoxy-PEG-hu apo-transferrin and hu apo-transferrin control were injected at time point 24h into 4 female mice (BALB/c) per group that had received 10 µg of Rabbit anti-methoxyPEG antibody (abcam, ab190652; conc. 0.1 mg/mL) at time point 0h by intravenous injection via the retro-orbital route (r.o.). Blood was collection every 24hrs as indicated on the X-axis.

Results are shown in Fig. 13. Free rabbit anti-PEG from serum was detected by standard ELISA using Methoxy-PEG-Biotin-coated streptavidin ELISA plates (CreativePEGworks); the Y-axis indicates relative anti-PEG decrease (in %) calculated from EC50 using GraphPad Prism, as in the other experiments.

This independent experiment corroborated the surprising results of Example 13, over a longer observation period. The 2 kDa PEG-SADC formulation was most effective and caused long lasting anti-PEG antibody lowering. In contrast, the 10kDa PEG-SADC formulation still achieved anti-PEG antibody lowering but was less effective.

### Non-patent references

Akbarzadehlaleh, Parvin, et al. "PEGylated human serum albumin: review of PEGylation, purification and characterization methods." Advanced pharmaceutical bulletin 6.3 (2016): 309.
Aldosari, Basmah N., Iman M. Alfagih, and Alanood S. Almurshedi. "Lipid nanoparticles as delivery systems for RNA- based vaccines." Pharmaceutics 13.2 (2021): 206.
Armstrong, Jonathan K. "The occurrence, induction, specificity and potential effect of antibodies against poly (ethylene glycol)." Pegylated protein drugs: Basic science and clinical applications. Birkhäuser Basel, 2009. 147-168.
Balakrishnan, Balaji, and Ernest David. "Biopolymers augment viral vectors based gene delivery." Journal of biosciences 44.4 (2019): 84.
Barry, Michael A., Jeffrey D. Rubin, and Shao-Chia Lu. "Retargeting adenoviruses for therapeutic applications and vaccines." FEBS letters (2020).
Binder, Uli, and Arne Skerra. "PASylation: a versatile technology to extend drug delivery." Current Opinion in Colloid & Interface Science 31 (2017): 10-17.
Booth, Claire, and H. Bobby Gaspar. "Pegademase bovine (PEG-ADA) for the treatment of infants and children with severe combined immunodeficiency (SCID)." Biologics: targets & therapy 3 (2009): 349.
Bozovičar, Krištof, and Tomaž Bratkovič. "Small and Simple, yet Sturdy: Conformationally Constrained Peptides with Remarkable Properties." International Journal of Molecular Sciences 22.4 (2021): 1611.
Carter, John Mark, and Larry Loomis-Price. "B cell epitope mapping using synthetic peptides." Current protocols in immunology 60.1 (2004): 9-4.
Dong, Yetian, et al. "A systematic review of SARS-CoV-2 vaccine candidates." Signal transduction and targeted therapy 5.1 (2020): 1-14.
Dijkstra, C. D., et al. "The heterogeneity of mononuclear phagocytes in lymphoid organs: distinct macrophage subpopulations in rat recognized by monoclonal antibodies ED1, ED2 and ED3." Microenvironments in the Lymphoid System. Springer, Boston, MA, 1985. 409-419.
Chapman, Andrew P. "PEGylated antibodies and antibody fragments for improved therapy: a review." Advanced drug delivery reviews 54.4 (2002): 531-545.
Elia, Natalie. "Using unnatural amino acids to selectively label proteins for cellular imaging: a cell biologist viewpoint." The FEBS Journal 288.4 (2021): 1107-1117. Epub 2020 Jul 22.
Elliott, Serra E., et al. "A pre-eclampsia-associated Epstein-Barr virus antibody cross-reacts with placental GPR50." Clinical Immunology 168 (2016): 64-71.
Erlandsson, Ann, et al. "In vivo clearing of idiotypic antibodies with antiidiotypic antibodies and their derivatives." Molecular immunology 43.6 (2006): 599-606.
Etzerodt, Anders, et al. "Efficient intracellular drug-targeting of macrophages using stealth liposomes directed to the hemoglobin scavenger receptor CD163." Journal of controlled release 160.1 (2012): 72-80.
Fabriek, Babs O., et al. "The macrophage scavenger receptor CD163 functions as an innate immune sensor for bacteria." Blood 113.4 (2009): 887-892.
Galanis, Kosmas A., et al. "Linear B-cell epitope prediction for in silico vaccine design: a performance review of methods available via command-line interface." International journal of molecular sciences 22.6 (2021): 3210.
Garay, Ricardo P., et al. "Antibodies against polyethylene glycol in healthy subjects and in patients treated with PEG-conjugated agents." (2012): 1319-1323.
Garces, Jorge Carlos, et al. "Antibody-mediated rejection: a review." The Ochsner Journal 17.1 (2017): 46.
Gaspar, Maria Manuela, et al. "Targeted delivery of transferrin-conjugated liposomes to an orthotopic model of lung cancer in nude rats." Journal of aerosol medicine and pulmonary drug delivery 25.6 (2012): 310-318.
Gazarian, Karlen, et al. "Mimotope peptides selected from phage display combinatorial library by serum antibodies of pigs experimentally infected with Taenia solium as leads to developing diagnostic antigens for human neurocysticercosis." Peptides 38.2 (2012): 381-388.
Gfeller, David, et al. "Current tools for predicting cancer-specific T cell immunity." Oncoimmunology 5.7 (2016): e1177691.
Granfeldt, Asger, et al. "Targeting dexamethasone to macrophages in a porcine endotoxemic model." Critical Care Medicine 41.11 (2013): e309-e318.
Graversen, Jonas H., et al. "Targeting the hemoglobin scavenger receptor CD163 in macrophages highly increases the anti-inflammatory potency of dexamethasone." Molecular Therapy 20.8 (2012): 1550-1558.
Gurda, Brittney L., et al. "Mapping a neutralizing epitope onto the capsid of adeno-associated virus serotype 8." Journal of virology 86.15 (2012): 7739-7751.
Hansen, Lajla Bruntse, Soren Buus, and Claus Schafer-Nielsen. "Identification and mapping of linear antibody epitopes in human serum albumin using high-density peptide arrays." PLoS One 8.7 (2013): e68902.
Hoang Thi, Thai Thanh, et al. "The Importance of Poly (ethylene glycol) Alternatives for Overcoming PEG Immunogenicity in Drug Delivery and Bioconjugation." Polymers 12.2 (2020): 298.
Homma, Masayuki, et al. "A Novel Fusion Protein, AChR-Fc, Ameliorates Myasthenia Gravis by Neutralizing Antiacetylcholine Receptor Antibodies and Suppressing Acetylcholine Receptor-Reactive B Cells." Neurotherapeutics 14.1 (2017): 191-198.
Howard Jr, James F. "Myasthenia gravis: the role of complement at the neuromuscular junction." Annals of the New York Academy of Sciences 1412.1 (2018): 113-128.
Howarth, M., & Brune, K. D. (2018). New routes and opportunities for modular construction of particulate vaccines: stick, click and glue. Frontiers in immunology, 9, 1432.
Inglut, Collin T., et al. "Immunological and toxicological considerations for the design of liposomes." Nanomaterials 10.2 (2020): 190.
Ishida, Tatsuhiro, et al. "Accelerated blood clearance of PEGylated liposomes upon repeated injections: effect of doxorubicin-encapsulation and high-dose first injection." Journal of controlled release 115.3 (2006): 251-258.
Jain, Swati, et al. "Nucleic acid therapeutics: a focus on the development of aptamers." Expert Opinion on Drug Discovery 16.3 (2021): 255-274.
Jansson, Liselotte, et al. "Immunotherapy With Apitopes Blocks the Immune Response to TSH Receptor in HLA-DR Transgenic Mice." Endocrinology 159.9 (2018): 3446-3457.
Jensen, Kamilla Kjærgaard, et al. "Improved methods for predicting peptide binding affinity to MHC class II molecules." Immunology 154.3 (2018): 394-406.
Jurtz, Vanessa, et al. "NetMHCpan-4.0: improved peptide-MHC class I interaction predictions integrating eluted ligand and peptide binding affinity data." The Journal of Immunology 199.9 (2017): 3360-3368.
Kang, Tse Siang, and Raymond C. Stevens. "Structural aspects of therapeutic enzymes to treat metabolic disorders." Human mutation 30.12 (2009): 1591-1610.
Kant, Sam, and Mohamed G. Atta. "Therapeutic advances in Fabry disease: The future awaits." Biomedicine & Pharmacotherapy 131 (2020): 110779.
Kaur, Simran Preet, and Vandana Gupta. "COVID-19 Vaccine: A comprehensive status report." Virus research (2020): 198114.
Kim, Jaesung, et al. "Enhancing the therapeutic efficacy of adenovirus in combination with biomaterials." Biomaterials 33.6 (2012): 1838-1850.
Kim, Tae Hyung, et al. "PEG-transferrin conjugated TRAIL (TNF-related apoptosis-inducing ligand) for therapeutic tumor targeting." Journal of controlled release 162.2 (2012): 422-428.
Ko 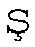 aloǧlu-Yal 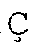 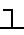 n, Zeynep, et al. "Predicting T cell recognition of MHC class I restricted neoepitopes." Oncoimmunology 7.11 (2018): e1492508.
Koniev, Oleksandr, and Alain Wagner. "Developments and recent advancements in the field of endogenous amino acid selective bond forming reactions for bioconjugation." Chemical Society Reviews 44.15 (2015): 5495-5551.
Krutzke, Lea, et al. "Substitution of blood coagulation factor X-binding to Ad5 by position-specific PEGylation: preventing vector clearance and preserving infectivity." Journal of Controlled Release 235 (2016): 379-392.
Lazaridis, Konstantinos, et al. "Specific removal of autoantibodies by extracorporeal immunoadsorption ameliorates experimental autoimmune myasthenia gravis." Journal of neuroimmunology 312 (2017): 24-30.
Lee, Gary K., et al. "PEG conjugation moderately protects adeno-associated viral vectors against antibody neutralization." Biotechnology and bioengineering 92.1 (2005): 24-34.
Leung, Nicki YH, et al. "Screening and identification of mimotopes of the major shrimp allergen tropomyosin using one-bead-one-compound peptide libraries." Cellular & molecular immunology 14.3 (2017): 308-318.
Lim, Sung In, and Inchan Kwon. "Bioconjugation of therapeutic proteins and enzymes using the expanded set of genetically encoded amino acids." Critical reviews in biotechnology 36.5 (2016): 803-815.
Lin, Chia-Hao, et al. "Identification of a major epitope by anti-interferon-γ autoantibodies in patients with mycobacterial disease." Nature medicine 22.9 (2016): 994.
Lorentz, Kristen M., et al. "Engineered binding to erythrocytes induces immunological tolerance to E. coli asparaginase." Science advances 1.6 (2015): e1500112.
Lu, Xueguang, and Ke Zhang. "PEGylation of therapeutic oligonucletides: From linear to highly branched PEG architectures." Nano research 11.10 (2018): 5519-5534.
Lubich, Christian, et al. "The mystery of antibodies against polyethylene glycol (PEG)-what do we know?." Pharmaceutical research 33.9 (2016): 2239-2249.
Luo, Jie, et al. "Main immunogenic region structure promotes binding of conformation-dependent myasthenia gravis autoantibodies, nicotinic acetylcholine receptor conformation maturation, and agonist sensitivity." Journal of Neuroscience 29.44 (2009): 13898-13908.
Luo, Jie, and Jon Lindstrom. "AChR-specific immunosuppressive therapy of myasthenia gravis." Biochemical pharmacology 97.4 (2015): 609-619.
Luo, Ying-Li, et al. "Macrophage-specific in vivo gene editing using cationic lipid-assisted polymeric nanoparticles." ACS nano 12.2 (2018): 994-1005.
Lutz, Gordon J., Shashank R. Sirsi, and Jason H. Williams. "PEG-PEI copolymers for oligonucleotide delivery to cells and tissues." Gene Therapy Protocols. Humana Press, 2008. 141-150.
Majowicz, Anna, et al. "Seroprevalence of pre-existing NABs against AAV1, 2, 5, 6 and 8 in the South African Hemophilia B patient population." (2019): 3353-3353.
Mazor, Ronit, et al. "Tolerogenic nanoparticles restore the antitumor activity of recombinant immunotoxins by mitigating immunogenicity." Proceedings of the National Academy of Sciences 115.4 (2018): E733-E742.
McSweeney, Morgan D., et al. "Overcoming anti-PEG antibody mediated accelerated blood clearance of PEGylated liposomes by pre-infusion with high molecular weight free PEG." Journal of Controlled Release 311 (2019): 138-146.
Meister, Daniel, S. Maryamdokht Taimoory, and John F. Trant. "Unnatural amino acids improve affinity and modulate immunogenicity: Developing peptides to treat MHC type II autoimmune disorders." Peptide Science 111.1 (2019): e24058.
Meneguetti, Giovanna Pastore, et al. "Novel site-specific PEGylated L-asparaginase." PloS one 14.2 (2019): e0211951.
Mingozzi, Federico, et al. "Overcoming preexisting humoral immunity to AAV using capsid decoys." Science translational medicine 5.194 (2013): 194ra92-194ra92.
Mingozzi, Federico, and Katherine A. High. "Overcoming the host immune response to adeno-associated virus gene delivery vectors: the race between clearance, tolerance, neutralization, and escape." Annual review of virology 4 (2017): 511-534.
Morimoto et. al., Bioconjugate Chemistry 25 (8) (2014): 1479-1491
Moussa, Ehab M., et al. "Immunogenicity of therapeutic protein aggregates." Journal of pharmaceutical sciences 105.2 (2016): 417-430.
Miller, Manuel M. "Post-translational modifications of protein backbones: unique functions, mechanisms, and challenges." Biochemistry 57.2 (2017): 177-185.
O'Riordan, Catherine R., et al. "PEGylation of adenovirus with retention of infectivity and protection from neutralizing antibody in vitro and in vivo." Human gene therapy 10.8 (1999): 1349-1358.
Park, Eun Ji, et al. "Emerging PEGylated non-biologic drugs." Expert opinion on emerging drugs 24.2 (2019): 107-119.
Peters, Bjoern, et al. "A community resource benchmarking predictions of peptide binding to MHC-I molecules." PLoS computational biology 2.6 (2006): e65.
Pishesha, Novalia, et al. "Engineered erythrocytes covalently linked to antigenic peptides can protect against autoimmune disease." Proceedings of the National Academy of Sciences (2017): 201701746.
Podust, Vladimir N., et al. "Extension of in vivo half-life of biologically active peptides via chemical conjugation to XTEN protein polymer." Protein Engineering, Design & Selection 26.11 (2013): 743-753.
Qiu, Yingshan, et al. "Effective mRNA pulmonary delivery by dry powder formulation of PEGylated synthetic KL4 peptide." Journal of Controlled Release 314 (2019): 102-115.
Ramana, Jayashree, and Kusum Mehla. "Immunoinformatics and Epitope Prediction." Immunoinformatics. Humana, New York, NY, 2020. 155-171.
Rey et al., Clinical Immunology 96 (3) (2000): 269-279
Ruff, Robert L., and Robert P. Lisak. "Nature and action of antibodies in myasthenia gravis." Neurologic clinics 36.2 (2018): 275-291.
Rummler, Silke, et al. "Current techniques for AB0-incompatible living donor liver transplantation." World journal of transplantation 6.3 (2016): 548.
Runcie, Karie, et al. "Bi-specific and tri-specific antibodies-the next big thing in solid tumor therapeutics." Molecular Medicine 24.1 (2018): 50.
Ryan, Brent J., Ahuva Nissim, and Paul G. Winyard. "Oxidative post-translational modifications and their involvement in the pathogenesis of autoimmune diseases." Redox biology 2 (2014): 715-724.
Saifer, Mark GP, et al. "Selectivity of binding of PEGs and PEG-like oligomers to anti-PEG antibodies induced by methoxyPEG-proteins." Molecular immunology 57.2 (2014): 236-246.
Sekiya, Toshiki, et al. "PEGylation of a TLR2-agonist-based vaccine delivery system improves antigen trafficking and the magnitude of ensuing antibody and CD8+ T cell responses." Biomaterials 137 (2017): 61-72.
Shanmugam, Arulkumaran, et al. "Identification of PSA peptide mimotopes using phage display peptide library." Peptides 32.6 (2011): 1097-1102.
Sharp, Kim A., et al. "Synthesis and application of a poly (ethylene glycol)-antibody affinity ligand for cell separations in aqueous polymer two-phase systems." Analytical biochemistry 154.1 (1986): 110-117.
Sherman, Merry R., et al. "Role of the methoxy group in immune responses to mPEG-protein conjugates." Bioconjugate chemistry 23.3 (2012): 485-499.
Shiraishi, Kouichi, and Masayuki Yokoyama. "Toxicity and immunogenicity concerns related to PEGylated-micelle carrier systems: a review." Science and technology of advanced materials 20.1 (2019): 324-336.
Siang Ong, Yong, et al. "Recent advances in synthesis and identification of cyclic peptides for bioapplications." Current topics in medicinal chemistry 17.20 (2017): 2302-2318.
Siekmann, Jürgen, and Peter L. Turecek. "PEGylation of human coagulation factor VIII and other plasma proteins." Polymer-Protein Conjugates. Elsevier, 2020. 155-174.
Skytthe, Maria K., Jonas Heilskov Graversen, and Søren K. Moestrup. "Targeting of CD163+ Macrophages in Inflammatory and Malignant Diseases." International Journal of Molecular Sciences 21.15 (2020): 5497.
Solomon, Melani, and Silvia Muro. "Lysosomal enzyme replacement therapies: Historical development, clinical outcomes, and future perspectives." Advanced drug delivery reviews 118 (2017): 109-134.
Sørensen, Karen Kristine, et al. "Liver sinusoidal endothelial cells." Comprehensive Physiology 5.4 (2011): 1751-1774.
Spiess, Christoph, Qianting Zhai, and Paul J. Carter. "Alternative molecular formats and therapeutic applications for bispecific antibodies." Molecular immunology 67.2 (2015): 95-106.
Suk, Jung Soo, et al. "PEGylation as a strategy for improving nanoparticle-based drug and gene delivery." Advanced drug delivery reviews 99 (2016): 28-51.
Sun, Pingping, et al. "Advances in in-silico B-cell epitope prediction." Current topics in medicinal chemistry 19.2 (2019): 105-115.
Swierczewska, Magdalena, Kang Choon Lee, and Seulki Lee. "What is the future of PEGylated therapies?." Expert opinion on emerging drugs 20.4 (2015): 531-536.
Sun, Yanping, et al. "Exploring the functions of polymers in adenovirus-mediated gene delivery: Evading immune response and redirecting tropism." Acta biomaterialia 97 (2019): 93-104.
Taddeo, Adriano, et al. "Selection and depletion of plasma cells based on the specificity of the secreted antibody." European journal of immunology 45.1 (2015): 317-319.
Teschner, Sven, et al. "ABO-incompatible kidney transplantation using regenerative selective immunoglobulin adsorption." Journal of clinical apheresis 27.2 (2012): 51-60.
Tetala, Kishore KR, et al. "Selective depletion of neuropathy-related antibodies from human serum by monolithic affinity columns containing ganglioside mimics." Journal of medicinal chemistry 54.10 (2011): 3500-3505.
Torres-Obreque, Karin, et al. "Production of a novel N-terminal PEGylated crisantaspase." Biotechnology and applied biochemistry 66.3 (2019): 281-289.
Turk, Viktorija Erdeljic. "Anaphylaxis associated with the mRNA COVID-19 vaccines: Approach to allergy investigation." Clinical Immunology 227 (2021): 108748.
Vincent, Angela, et al. "Serological and experimental studies in different forms of myasthenia gravis." Annals of the New York Academy of Sciences 1413.1 (2018): 143-153.
Wallukat, Gerd, et al. "Patients with preeclampsia develop agonistic autoantibodies against the angiotensin AT 1 receptor." The Journal of clinical investigation 103.7 (1999): 945-952.
Weaver, Eric A., and Michael A. Barry. "Effects of shielding adenoviral vectors with polyethylene glycol on vector-specific and vaccine-mediated immune responses." Human gene therapy 19.12 (2008): 1369-1382.
Wegmann, Frank, et al. "Polyethyleneimine is a potent mucosal adjuvant for viral glycoprotein antigens." Nature biotechnology 30.9 (2012): 883-888.
Worm, Margitta, et al. "Practical recommendations for the allergological risk assessment of the COVID-19 vaccination-a harmonized statement of allergy centers in Germany." Allergologie select 5 (2021): 72.
Yang, Qi, and Samuel K. Lai. "Anti-PEG immunity: emergence, characteristics, and unaddressed questions." Wiley Interdisciplinary Reviews: Nanomedicine and Nanobiotechnology 7.5 (2015): 655-677.
Yang, Qi, et al. "Analysis of pre-existing IgG and IgM antibodies against polyethylene glycol (PEG) in the general population." Analytical chemistry 88.23 (2016): 11804-11812.
Yoshimoto, Noriko, et al. "PEG chain length impacts yield of solid-phase protein PEGylation and efficiency of PEGylated protein separation by ion-exchange chromatography: Insights of mechanistic models." Biotechnology journal 8.7 (2013): 801-810.
Zhang, Tingting, et al. "Moderate PEGylation of the carrier protein improves the polysaccharide-specific immunogenicity of meningococcal group A polysaccharide conjugate vaccine." Vaccine 33.28 (2015): 3208-3214.
Zhou, Cissy C., et al. "Angiotensin receptor agonistic autoantibodies induce pre-eclampsia in pregnant mice." Nature medicine 14.8 (2008): 855.
Zinsli, Léa V., et al. "Deimmunization of protein therapeutics-Recent advances in experimental and computational epitope prediction and deletion." Computational and Structural Biotechnology Journal (2020).

## Claims

1. A compound comprising
- a biopolymer scaffold, and
- one or more polyethylene glycol (PEG) chains.

2. The compound of claim 1, wherein said one or more PEG chains comprise at least two PEG chains, each of the two PEG chains having a molecular weight of 100-10000 Da, preferably 200-8000 Da, more preferably 300-6000 Da, yet more preferably 400-5000 Da, even more preferably 500-4000 Da, yet even more preferably 600-3000 Da, especially 700-2500 Da.

3. The compound of claim 2, wherein said molecular weight is 1500-2500 Da.

4. The compound of any one of claims 1 to 3, wherein said one or more PEG chains comprise at least one PEG chain having a free methoxy end group or a free hydroxyl end group.

5. The compound of any one of claims 1 to 4, wherein the biopolymer scaffold is selected from the group consisting of alpha1-globulins, alpha2-globulins, beta-globulins and albumin, in particular wherein the biopolymer scaffold is haptoglobin or transferrin, especially transferrin; or wherein the biopolymer scaffold is an antibody specific for a CD163 protein, or a CD163-binding fragment thereof.

6. The compound of any one of claims 1 to 5, wherein at least a portion of the one or more PEG chains are covalently bound to the biopolymer scaffold via at least one linker, wherein the linker comprises a peptide or a single amino acid such as a cysteine.

7. The compound of any one of claims 1 to 6, wherein the compound is non-immunogenic in a mammal, preferably in a human, in a non-human primate, in a sheep, in a pig, in a dog or in a rodent.

8. A pharmaceutical composition comprising the compound of any one of claims 1 to 7 and at least one pharmaceutically acceptable excipient.

9. The pharmaceutical composition of claim 8, wherein the composition is non-immunogenic in humans.

10. The pharmaceutical composition of claim 8 or 9 for use in therapy.

11. The pharmaceutical composition for use according to claim 10, for use in inhibiting an immune reaction of an individual to a treatment with an active agent, wherein the active agent comprises at least one PEG, in particular wherein the active agent is PEGylated; preferably wherein the pharmaceutical composition is administered at least twice within a 96-hour window, wherein the window is followed by administration of the active agent within 24 hours.

12. The pharmaceutical composition for use according to claim 10, for use in inhibiting neutralization, in particular accelerated blood clearance, of an active agent in an individual, wherein the active agent comprises at least one PEG, in particular wherein the active agent is PEGylated; preferably wherein the pharmaceutical composition is administered at least twice within a 96-hour window, wherein the window is followed by administration of the active agent within 24 hours.

13. The pharmaceutical composition for use according to claim 11 or 12, wherein the active agent is a protein or peptide, preferably wherein the active agent is selected from the group of enzymes, enzyme inhibitors, antibodies, antibody fragments, antibody mimetics, antibody-drug conjugates, hormones, growth factors, clotting factors and cytokines; or wherein the active agent is a viral vector such as a viral vector for gene therapy or vaccination.

14. The pharmaceutical composition for use according to any one of claims 11 or 12, wherein the active agent is a nucleic acid-lipid particle, a nucleic acid-polymer particle, a nucleic acid-lipid-polymer particle, or a nucleic acid; preferably wherein the nucleic acid is RNA, in particular mRNA or siRNA, or DNA.

15. A pharmaceutical composition, comprising the compound of any one of claims 1 to 7 and further comprising an active agent and optionally at least one pharmaceutically acceptable excipient,
wherein the active agent comprises at least one PEG, in particular wherein the active agent is PEGylated;
preferably wherein the active agent is a viral vector or a protein or peptide, especially selected from the group of enzymes, enzyme inhibitors, antibodies, antibody fragments, antibody mimetics, antibody-drug conjugates, hormones, growth factors, clotting factors and cytokines; and/or preferably wherein the active agent is a nucleic acid-lipid particle, a nucleic acid-polymer particle, a nucleic acid-lipid-polymer particle, or a nucleic acid, in particular wherein the nucleic acid is RNA, preferably mRNA or siRNA, or DNA.
